# EUROPEAN PATENT APPLICATION

(11) **EP 3 112 458 A1**
(43) Date of publication of application: **04.01.2017**
(21) Application number: 15174342.4
(22) Date of filing: 29.06.2015
(51) Int. Cl.: C12N 9/10, C07K 14/34, C07K 14/345

(54) **MICROBIOLOGICAL PRODUCTION OF SHORT FATTY ACIDS AND USES THEREOF**

(71) Applicant: Johann Wolfgang Goethe-Universität Frankfurt am Main, 60323 Frankfurt am Main (DE)
(72) Inventor: Grininger, Martin, 60316 Frankfurt (DE); Gajewski, Jan, 60316 Frankfurt (DE); Boles, Eckard, 64289 Darmstadt (DE)
(74) Representative: Grahn, Sibylla Maria

(57) **Abstract**

The present invention relates to proteins involved in fatty acid synthesis, such as fatty acid synthases (FAS) variants, comprising one or more polypeptide chains, wherein said polypeptide chain(s) comprise one or more subunits comprising a malonyl/palmitoyl transferase domain (MPT domain), acetyl transferase domain (AT domain), and/or ketoacyl synthase domain (KS domain), and at least one amino acid substitution in the MPT domain at a position corresponding to R90, in the AT domain at a position corresponding to I151, and/or in the KS domain, preferably in the acyl binding channel, to modulate affinities of acyl intermediates, and optionally further amino acid substitution(s). The present invention relates to the respective polypeptide domains.

The present invention further relates to nucleic acid molecules encoding the proteins (or the polypeptide domains) and to host cells containing said nucleic acid molecules. The present invention further relates to a method for the production of short fatty acids, CoA esters of short fatty acids, ethyl esters of short fatty acids, esters of short fatty acids with other metabolites, and/or enzyme bound short fatty acids (C₆ to C₁₂), comprising the expression of said nucleic acid molecules, preferably in said host cells. The present invention further relates to a method for the production of biofuels, flavoring compounds and/or fine chemicals, comprising the expression of said nucleic acid molecules, preferably in said host cells. The present invention also relates to the use of the proteins, nucleic acids molecule or host cells for the production of short fatty acids (C₆ to C₁₂), the production of CoA esters of short fatty acids (C₆ to C₁₂), the production of ethyl esters of short fatty acids (C₆ to C₁₂), the production of esters of short fatty acids (C₆ to C₁₂) with other metabolites , the production of enzyme bound short fatty acids (C₆ to C₁₂), the production of biofuels, fine chemicals and/or flavoring substances.

## Description

The present invention relates to proteins involved in fatty acid synthesis, such as fatty acid synthases (FAS) variants, comprising one or more polypeptide chains, wherein said polypeptide chain(s) comprise one or more subunits comprising a malonyl/palmitoyl transferase domain (MPT domain), acetyl transferase domain (AT domain), and/or ketoacyl synthase domain (KS domain), and at least one amino acid substitution in the MPT domain at a position corresponding to R90, in the AT domain at a position corresponding to I151, and/or in the KS domain, preferably in the acyl binding channel, to modulate affinities of acyl intermediates, and optionally further amino acid substitution(s). The present invention relates to the respective polypeptide domains.

The present invention further relates to nucleic acid molecules encoding the proteins (or the polypeptide domains) and to host cells containing said nucleic acid molecules. The present invention further relates to a method for the production of short fatty acids, CoA esters of short fatty acids, ethyl esters of short fatty acids, esters of short fatty acids with other metabolites, and/or enzyme bound short fatty acids (C₆ to C₁₂), comprising the expression of said nucleic acid molecules, preferably in said host cells. The present invention further relates to a method for the production of biofuels, flavoring compounds and/or fine chemicals, comprising the expression of said nucleic acid molecules, preferably in said host cells. The present invention also relates to the use of the proteins, nucleic acids molecule or host cells for the production of short fatty acids (C₆ to C₁₂), the production of CoA esters of short fatty acids (C₆ to C₁₂), the production of ethyl esters of short fatty acids (C₆ to C₁₂), the production of esters of short fatty acids (C₆ to C₁₂) with other metabolites , the production of enzyme bound short fatty acids (C₆ to C₁₂), the production of biofuels, fine chemicals and/or flavoring substances.

### BACKGROUND OF THE INVENTION

With the rising demand for industrial products from a growing world population, the call for chemicals from renewable sources has become louder. Especially the efforts have been intensified, where fatty acids (FA) can play a crucial role as a platform chemical in the production of fine chemicals or even to replace fossil derived fuel by biofuels (Runguphan & Keasling, 2014; Choi & Lee, 2013). Accordingly, the production of FA in microorganisms has been investigated extensively, constantly pushing the limits: Yields of *in vivo* production have been shown in *S. cerevisiae* of up to 400 mg/L free FA (Runguphan & Keasling, 2014) and more recently even 2.2 g/L (Leber et al., 2015) (in both cases products were mostly in the long chain range); and up to 4.8 g/L in *E. coli* (predominantly C₁₄ and C₁₆) (Liu et al., 2012).

In microorganisms themselves, FA serve several purposes, mainly as a part of membranes, in signaling but also in energy storage. Their de-novo production is tightly regulated and conducted by an enzyme group, the fatty acid synthases (FASs) (Tehlivets et al., 2007). Throughout all organisms, their reaction mechanisms and their chemistry are essentially the same: A ketoacyl synthase (KS) is responsible for the elongation of an acyl chain starter molecule, typically an acetyl-CoA, with malonyl. The resulting β-ketoacyl intermediate is then processed in a series of reaction steps, in the ketoacyl reductase (KR), the dehydratase (DH) and enoyl reductase (ER), to a fully reduced acyl chain. This acyl chain which is now extended by two carbons, serves as a starter for the next cycle. The process repeats itself until the final product is cleft off.

As far as the overall structural organization of fatty acid synthases (FASs) is concerned, two types are distinguished: In type I FASs, all necessary enzymatic functions of fatty acid (FA) production are concentrated in one multienzymatic complex, whereas in type II FAS systems, each reaction is catalyzed by a separate enzyme. For type I FAS systems, the intermediates are always covalently bound to the multienzymatic complex leading to extremely high efficiency. Type II FASs are found in bacteria, while type I FASs are typical for all eukaryotic organisms and few actinobacteria, among these also *Corynebacterium ammoniagenes.*

In detail, in the *C*. *ammoniagenes* FAS, one set of domains is distributed on one polypeptide chain in the following order (starting from the N-terminus): the AT domain, the ER domain, the DH domain, the MPT domain, the ACP domain, the KR domain and the KS domain. Multiple copies of the corresponding polypeptide chain form the homohexameric 1.9 MDa complex. Structural data from bacterial FAS (to which class the *C*. *ammoniagenes* FAS belongs) have documented high homology to the fungal FAS systems (such as from *S*. *cerevisiae*) (Boehringer 2013). The FAS from *S. cerevisiae* has been object of extensive x-ray structural analysis with resolutions up to 3.1 Å (see e.g. Jenni et al., 2007; Johansson et al., 2008). Its interpretation has led to substantially new insights in the reaction mechanisms of the whole FAS enzyme family (Beld et al., 2015).

The product distribution of the C. *ammoniagenes* FAS is naturally in the long chain range of C₁₆-CoA and C₁₈-CoA (Kawaguchi et al., 1980) and not directly suitable for applications where short FA are needed, as for instance biofuels in the petrol range. For this purpose, products typically have to have a length of C₄ to C₁₂ (Peralta-Yahya et al., 2012). Previous engineering efforts for the production of short FA have heavily relied on the utilization of thioesterases (TEs) with known specificities for short chain products (Beld et al., 2015), e.g. in a proof of principle study for the alkane production in *E. coli* (Choi & Lee, 2013) or the production of the precursors, short FA, in *S. cerevisiae* (with total yields up to 111 mg/L) (Leber et al., 2014). In contrast, the rational engineering for the production of short FA was believed to be hard to achieve (Beld et al., 2015; Leber et al., 2014).

US 2003/0145350 A1 discloses DNA sequences which code for a protein having the enzymatic activity of a beta-ketoacyl ACP synthase (KAS) of the enzyme complex of the fatty acid synthase (FAS). US 2003/0145350 A1 further discloses transgenic plants and micro-organisms which containing said nucleic acid sequences and a method for influencing the fatty acid pattern and/or for increasing the fatty acid content, especially the content of short and middle chain fatty acids, in plants, especially in seed tissues that synthesize and/or store triacylglycerols, as well as in micro-organisms, especially bacteria and algae. The method comprises the expression of proteins having the activity of a KAS of the enzyme complex or the fatty acid synthase in transgenic plants or micro-organisms.

There is a need in the art for further as well as improved means and methods for producing fatty acids enzymatically in a controlled manner (for example in microorganisms), in particular fatty acids that are suitable for biofuel production, fine chemicals and other compounds.

### SUMMARY OF THE INVENTION

According to the present invention this object is solved by a polypeptide or protein involved in fatty acid synthesis, said polypeptide or protein comprising one or more polypeptide chains, wherein said polypeptide chain(s) comprise
said protein comprising one or more polypeptide chains, wherein said polypeptide chain(s) comprise
(i) one or more subunits comprising the amino acid sequences of
   SEQ ID NO: 1 (malonyl/palmitoyl transferase domain, MPT domain);
   SEQ ID NO: 2 (acetyl transferase domain, AT domain), and/or
   SEQ ID NO: 3 (ketoacyl synthase domain, KS domain);
(ii) at least one amino acid substitution
   - in the MPT domain at a position corresponding to R90 of the amino acid sequence of SEQ ID NO: 1;
   - in the AT domain at a position corresponding to I151 of the amino acid sequence of SEQ ID NO: 2;
      and/or
   - in the KS domain, preferably in the acyl binding channel, preferably selected from a position corresponding to G227, M228 and L256 of the amino acid sequence of SEQ ID NO: 3;
wherein the amino acid sequence comprising the at least one amino acid substitution has at least 50%, or preferably at least 60% or 70% or 80% or 90% or 95% sequence identity to the respective amino acid sequence of SEQ ID NO: 1, 2 and/or 3.

According to the present invention this object is solved by a polypeptide domain comprising
(i) one or more subunits comprising the amino acid sequences of
   SEQ ID NO: 1 (malonyl/palmitoyl transferase domain, MPT domain);
   SEQ ID NO: 2 (acetyl transferase domain, AT domain), or
   SEQ ID NO: 3 (ketoacyl synthase domain, KS domain);
(ii) at least one amino acid substitution
   - in the MPT domain at a position corresponding to R90 of the amino acid sequence of SEQ ID NO: 1;
   - in the AT domain at a position corresponding to I151 of the amino acid sequence of SEQ ID NO: 2;
      and/or
   - in the KS domain, preferably in the acyl binding channel, preferably selected from a position corresponding to G227, M228 and L256 of the amino acid sequence of SEQ ID NO: 3;
wherein the amino acid sequence comprising the at least one amino acid substitution has at least 50%, or preferably at least 60% or 70% or 80% or 90% or 95% sequence identity to the respective amino acid sequence of SEQ ID NO: 1, 2 and/or 3.

According to the present invention this object is solved by a nucleic acid molecule coding for a protein of the present invention or a polypeptide domain of the present invention.

According to the present invention this object is solved by a host cell, containing a nucleic acid molecule of the present invention and preferably expressing said nucleic acid molecule, wherein said host cell is preferably selected from a bacterial cell or a fungus cell, more preferably a yeast cell, or an algae cell.

According to the present invention this object is solved by a method for the production of short fatty acids, CoA esters of short fatty acids, ethyl esters of short fatty acids, esters of short fatty acids with other metabolites, and/or enzyme bound short fatty acids (C₆ to C₁₂), comprising the expression of a nucleic acid molecule according to the present invention, preferably in a host cell according to the present invention.

According to the present invention this object is solved by a method for the production of biofuels, flavoring compounds and/or fine chemicals comprising the expression of a nucleic acid molecule according to the present invention, preferably in a host cell according to the present invention.

According to the present invention this object is solved by using a protein or polypeptide according to the present invention, a nucleic acid molecule according to the present invention, or a host cell according to the present invention for the production of short fatty acids (C₆ to C₁₂).

According to the present invention this object is solved by using a protein or polypeptide according to the present invention, a nucleic acid molecule according to the present invention, or a host cell according to the present invention for the production of CoA esters of short fatty acids (C₆ to C₁₂).

According to the present invention this object is solved by using a protein or polypeptide according to the present invention, a nucleic acid molecule according to the present invention, or a host cell according to the present invention for the production of ethyl esters of short fatty acids (C₆ to C₁₂) or esters of short fatty acids (C₆ to C₁₂) with other metabolites.

According to the present invention this object is solved by using a protein or polypeptide according to the present invention, a nucleic acid molecule according to the present invention, or a host cell according to the present invention for the production of enzyme bound short fatty acids (C₆ to C₁₂).

According to the present invention this object is solved by using a protein or polypeptide according to the present invention, a nucleic acid molecule according to the present invention, or a host cell according to the present invention for the production of biofuels, such as short alkanes, short alkenes, short alkynes, methyl ketones, short esters and/or alcohols.

According to the present invention this object is solved by using a protein or polypeptide according to the present invention, a nucleic acid molecule according to the present invention, or a host cell according to the present invention for the production of fine chemicals, such as natural compounds where preferably short fatty acids (C₆ to C₁₂) or their derivatives (such as CoA esters, methyl/ethyl esters, esters with other metabolites, enzyme bound fatty acids, alcohols) are used as building block(s).

According to the present invention this object is solved by using a protein or polypeptide according to the present invention, a nucleic acid molecule according to the present invention, or a host cell according to the present invention for the production of flavoring substances, such as esters from short fatty acids (C₆ to C₁₂).

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

### Fatty acid synthase variants and domains

As discussed above, the present invention provides fatty acid synthase (FAS) variants, in particular type I fatty acid synthase (type I FAS) variants.

The polypeptides or proteins of the present invention comprise one or more polypeptide chains. Said one or more polypeptide chains comprise one or more subunits comprising a malonyl/palmitoyl transferase domain (MPT domain), an acetyl transferase domain (AT domain), and/or a ketoacyl synthase domain (KS domain).

The invention provides a protein or polypeptide involved in fatty acid synthesis, preferably having fatty acid synthase activity.

The invention also provides polypeptide domain(s) which are involved in fatty acid synthesis activity, such as subunits comprising a malonyl/palmitoyl transferase domain (MPT domain), an acetyl transferase domain (AT domain), and/or a ketoacyl synthase domain (KS domain).

Fatty acid synthases or polypeptides/proteins involved in fatty acid synthesis comprise one or more polypeptide chains, such as two, three, four or more polypeptide chains.

Fatty acid synthases or polypeptides/proteins involved in fatty acid synthesis furthermore comprise several different catalytic domains or subunits. Said catalytic domains or subunits can be located on said different / one or more polypeptide chains. The catalytic domains or subunits can also be split in parts and the different parts can be located on said different / one or more polypeptide chains.

According to the invention, the polypeptides or proteins of the present invention further comprise at least one amino acid substitution in the MPT domain at a position corresponding to R90, in the AT domain at a position corresponding to I151, and/or in the KS domain, preferably in the acyl binding channel, to modulate affinities of acyl intermediates, and optionally further amino acid substitution(s).

In particular, the present invention provides a polypeptide or protein involved in fatty acid synthesis, said polypeptide comprising one or more polypeptide chains, said polypeptide chain(s) comprising
(i) one or more subunits comprising the amino acid sequences of
   SEQ ID NO: 1 (malonyl/palmitoyl transferase domain, MPT domain);
   SEQ ID NO: 2 (acetyl transferase domain, AT domain), and/or
   SEQ ID NO: 3 (ketoacyl synthase domain, KS domain);
(ii) at least one amino acid substitution
   - in the MPT domain at a position corresponding to R90 of the amino acid sequence of SEQ ID NO: 1;
   - in the AT domain at a position corresponding to I151 of the amino acid sequence of SEQ ID NO: 2;
      and/or
   - in the KS domain, preferably in the acyl binding channel, preferably selected from a position corresponding to G227, M228 and L256 of the amino acid sequence of SEQ ID NO: 3.

According to the present invention, the amino acid sequence comprising the at least one amino acid substitution has at least 50% or preferably at least 60% or 70% or 80% or 90% or 95% sequence identity to the respective amino acid sequence of SEQ ID NO: 1, 2 and/or 3.

In particular, the present invention provides a polypeptide domain comprising
(i) one or more subunits comprising the amino acid sequences of
   SEQ ID NO: 1 (malonyl/palmitoyl transferase domain, MPT domain);
   SEQ ID NO: 2 (acetyl transferase domain, AT domain), or
   SEQ ID NO: 3 (ketoacyl synthase domain, KS domain);
(ii) at least one amino acid substitution
   - in the MPT domain at a position corresponding to R90 of the amino acid sequence of SEQ ID NO: 1;
   - in the AT domain at a position corresponding to I151 of the amino acid sequence of SEQ ID NO: 2;
      and/or
   - in the KS domain, preferably in the acyl binding channel, preferably selected from a position corresponding to G227, M228 and L256 of the amino acid sequence of SEQ ID NO: 3;

According to the present invention, the amino acid sequence comprising the at least one amino acid substitution has at least 50%, or preferably at least 60% or 70% or 80% or 90% or 95% sequence identity to the respective amino acid sequence of SEQ ID NO: 1, 2 and/or 3,

In a preferred embodiment, the MPT and/or AT domain of the polypeptide(s) or protein(s) (or the polypeptide domain(s)) have an *in vitro* and/or *in vivo* transferase activity; and/or the KS domain of the polypeptide(s) or protein(s) has an *in vitro* and/or *in vivo* ketoacyl synthase activity.
This means that even though there is at least one amino acid substitution in the MPT domain, AT domain and/or KS domain, as disclosed herein, the MPT and/or AT domain of the polypeptide(s) or protein(s) have *an in vitro* and/or *in vivo* transferase activity; and/or the KS domain of the polypeptide(s) or protein(s) has an *in vitro* and/or *in vivo* ketoacyl synthase activity.

There are two principal classes of fatty acid synthases.
Type I systems utilize a single large, multifunctional enzyme and are common to both mammals and fungi (although the structural arrangement of fungal and mammalian synthases differ). A Type I fatty acid synthase system is also found in the CMN group of bacteria (Corynebacteria, mycobacteria, and nocardia). In these bacteria, the FAS I system produces palmititic acid, and can cooperate with the FAS II system to produce a greater diversity of lipid products. Type II is found in archaea and bacteria, and is characterized by the use of discrete, monofunctional enzymes for fatty acid synthesis.
For example, mammalian FAS usually consists of a homodimer of two identical protein subunits, in which three catalytic domains in the N-terminal section (-ketoacyl synthase (KS), malonyl/acetyltransferase (MAT), and dehydratase (DH)), are separated by a core region of 600 residues from four C-terminal domains (enoyl reductase (ER), -ketoacyl reductase (KR), acyl carrier protein (ACP) and thioesterase (TE)).

In one embodiment, the protein or polypeptide of the present invention is a type I FAS, such as type I FAS of *Corynebacterium ammoniagenes,* in particular FAS of *Corynebacterium ammoniagenes* with Uniprot Accession No. D5NXL2.

The amino acid sequence of FAS of *Corynebacterium ammoniagenes* of Uniprot Accession No. D5NXL2 is shown in SEQ ID NO. 7.

In one embodiment, the protein or polypeptide of the present invention is the type I FAS of *Corynebacterium ammoniagenes.*

In *C*. *ammoniagenes*, the chain for the type I FAS with Uniprot Accession No. D5NXL2 are encoded by gene *HMPREF0281_01212* (to be found in Genbank: ADNS01000009.1 at position 39376 - 48426).

SEQ ID NO: 1 shows the amino acid sequence of the MPT domain (part of sequence of Uniprot Identifier: D5NXL2).

Malonyl/palmitoyl transferase (MPT domain), (part of Uniprot Identifier: D5NXL2, residues 1319 - 1665)

SILEIVRNNPPLVVVDGEEFTHPDGTLFLTQFTQVAMATLGCAQIAELAEAGVKSQDTYFAG
HSVGEYNALAAYAQVLSLEAVVEIVYARGLTMHRLVDRDAEGNSNYGLAALRPNKIGIRAED
VFDYVAQVSQTRGEFLEIVNYNIAGVQYAVAGTRAGLAALAADAESRAPGMRSFIMIPGIDV
PFHSSHLLGGVDNFRTHLDMLIPDDVDLDILRGRYIPNLVARPFELTREFVEAMADVVDSTY
VNDILADFDKATDDPAKMGRTLLIELLAWQFASPVRWIETQDLLLSAPSVSAGAGTAGLGVE
RFVEIGVGSSPTLANMLGQTLRLPQYAGNPIEVLNVE

SEQ ID NO: 2 shows the amino acid sequence of the AT domain (part of sequence of Uniprot Identifier: D5NXL2).

Acetyl transferase (AT domain), (part of Uniprot Identifier: D5NXL2, residue 1 - 349)

MTIGISNHRLVNRLESEKFALSFSGQGFSWLPTLRTVIAGGNGAQVAGAVDAAGKLLEPVAD

ELVAALPHGFDPVEWAGNAEDPAWDLSDSSISTPGIVVAQYGLIESLKAQGLDPADAVAHIG

HSQGALATYISSGRAQAAEVIALAQLIGVAISKTARATGLIRIAAGSPMVSIAGISREQLAK

VLAKTGVDSVIGLQNNHNTFVVTGSPEDNNKVIEAIEKLAAKDAKAIENKERGGNPFAPRIN

ALPVQSGYHHPKMSAAVEMVTSWAERIGLDKDLAHDGAEAVLTEIVNWPATVQSAVDAGVTW
ILECGPEKGMAALTKAVVAGQGIGVQVVSTDEGQAALFD

SEQ ID NO: 3 shows the amino acid sequence of the KS domain (part of sequence of Uniprot Identifier: D5NXL2).

Ketoacyl synthase (KS domain), (part of Uniprot Identifier: D5NXL2, residues 2373 - 2930)

LDDMVVIVGAGEIGPVGSSRTRFEVEMTGDLTAAGVVELAWTTGLIAWDDASGAWFDAEGEE
IAEEDIYDRFHDEVLANVGVRKYHDDYGPKMPMVDNLAPELTTIYLERDMTFAVADEETART
FVADVEGATAHFDGEEWQVTRPAGAEVRVPRRVAMTRFVGGQIPQGFDPGVYGIPADMIDNL
DRVALWNLVCTVDAFLAAGFSPAELLAHVHPARVSSTQGTGMGGMQSMRSLYLDKLLAQPRP
NDILQEALPNVVAAHVMQSYVGGYGQMVHPVAACATAAVSVEEGMDKLRLRKADFVVAGGID
DLSIEGITGFGDMAATADSGEMEAKGIDHKYFSRANDRRRGGFIESEGGGTILLARGSLAAE
LGLPVLGVVGFAESFADGAHTSIPAPGLGALSAARGGADSRLAQGLAELGVSANEIAVISKH
DTSTNANDPNESDLHERIAASMGRDAGNPLFIISQKTLTGHAKGGAAAFQLVGLTQVLRNGV
LPPNRSLDCVDPALRQHTHLTWLREQLDLRATPPKAGLVTSLGFGHVSALVAVVHPGAFVEA

As far as the overall structural organization of fatty acid synthases (FASs) is concerned, two types are distinguished: In type I FASs, all necessary enzymatic functions of fatty acid (FA) production are concentrated in one multienzymatic complex, whereas in type II FAS systems, each reaction is catalyzed by a separate enzyme. For type I FAS systems, the intermediates are always covalently bound to the multienzymatic complex leading to extremely high efficiency. Type II FASs are found in bacteria, while type I FASs are typical for all eukaryotic organisms and few actinobacteria, among these also *C*. *ammoniagenes*.

In detail, in the *C*. *ammoniagenes* FAS, one set of domains is distributed on one polypeptide chain in the following order (starting from the N-terminus): the AT domain, the ER domain, the DH domain, the MPT domain, the ACP domain, the KR domain and the KS domain. Multiple copies of the corresponding polypeptide chain form the homohexameric 1.9 MDa complex. Structural data from bacterial FAS (to which class the *C. ammoniagenes* FAS belongs) and fungal FAS (such as from *S. cerevisiae*) systems have documented high homology between the two (Boehringer 2013). The FAS from *S. cerevisiae* has in particular been object of extensive X-ray structural analysis with resolutions up to 3.1 Å (see e.g. Jenni et al., 2007; Johansson et al., 2008). Its interpretation has led to substantially new insights in the reaction mechanisms of the whole FAS enzyme family (Beld et al., 2015).

As used herein, the term "at a position corresponding to" means the respective position in SEQ ID No: 1, 2 or 3 which, however, in related polypeptide chains can have another relative position number. The equivalent substitution can be determined by comparing a position in both sequences, which may be aligned for the purpose of comparison. The relative position of the amino acid can vary due to different length of the related polypeptide, or deletions or additions of amino acids in the related polypeptide.

**Table 1: List of mutations in the used C. ammoniagenes FAS and their equivalent in selected similar fatty acid synthases.**

| For the production of short fatty acids, the MPT, AT and KS domain were mutated. These mutations are listed below (*Corynebacterium ammoniagenes*, D5NXL2). The positions marked with # are numbered as used in the text (always in reference to SEQ ID NO. 1 for the MPT domain, SEQ ID NO. 2 for the AT domain and SEQ ID NO. 3 for the MPT domain). Position numbers marked by * are the same positions but numbering according to the whole polypeptide chain as described in the Uniprot Accession Number. The concept of mutations can be applied to various FAS systems with high homology to the used C. *ammoniagenes* FAS (for a more extensive list, see Table 2). For a selection of these systems, the positions are shown here (numbering according to the whole enzyme as described in the Uniprot Accession Numbers - the same way as for *C*. *ammonigenes* FAS numbering marked with *). Mutations in the *S*. *cerevisiae* FAS are subject to EP patent application 15 162 192.7 filed on April 1st, 2015). | | | | | | | | | | |
|---|---|---|---|---|---|---|---|---|---|---|
| Organisms | Uniprot | similarity overall | similarity MPT | similarity AT | similarity KS | MPT | AT | KS | | |
| **Corynebacterium ammoniagenes** | **D5NXL2** | | | | | **R90K^{#}** | **I151A^{#}** | **G227S^{#}** | **M228W^{#}** | **L258Y^{#}** |
| | (CamFAS, used in this study) | | | | | **R1408K*** | **I151A*** | **G2599S*** | **M2600W*** | **L2628Y*** |
| | | | | | | | | | | |
| Saccharomyces cerevisiae | P07149 /P19097 | 22.2% | 40.4% | 23.0% | 34.1% | R1834K | I1306A | G1250S | G1251M | F1279Y |
| | | | | | | | | | | |
| Corynebacterium ammoniagenes | Q59497 | 43.3% | 52.2% | 27.1% | 59.9% | R1425K | M150A | G2637S | I2638W | L2533Y |
| Corynebacterium glutamicum | Q6M2X6 | 62.4% | 75.3% | 47.0% | 74.3% | R1415K | I154A | G2586S | M2587W | L2637Y |
| Corynebacterium glutamicum | I0LHP5 | 44.1% | 51.4% | 25.8% | 61.2% | R1395K | I146A | G2564S | I2563W | L2593Y |
| | | | | | | | | | | |
| Corynebacterium casei | G7HWL7 | 87.9% | 91.4% | 83.4% | 95.9% | R1414K | I151A | G2614S | M2615W | L2633Y |
| Corynebacterium efficiens | Q8FMV7 | 63.1% | 74.8% | 48.4% | 62.0% | R1424K | I154A | G2611S | M2612W | L2640Y |
| Corynebacterium pseudotuberculosis | D9QC32 | 61.0% | 71.9% | 42.7% | 76.4% | R1438K | I158A | G2621S | L2622W | L2650Y |
| | | | | | | | | | | |
| Rhodococcus rhodochrous | W4A6R5 | 52.5% | 58.9% | 35.8% | 65.9% | R1479K | I192A | G2670S | M2671W | L2699Y |
| | | | | | | | | | | |
| Nocardia farcinica | Q5Z0D9 | 51.8% | 57.5% | 35.5% | 68.8% | R1502K | I199A | G2697S | M2698W | L2726Y |
| Nocardia asteroides | U5EKQ0 | 51.5% | 58.1% | 34.1% | 65.7% | R1489K | I185A | G2684S | M2685W | L2713Y |
| | | | | | | | | | | |
| Mycobacterium smegmatis | A0R1H7 | 50.8% | 54.3% | 35.4% | 65.2% | R1480K | I185A | G2685S | M2686W | L2714Y |
| Mycobacterium bovis | Q7TYD8 | 49.9% | 54.6% | 34.4% | 61.8% | R1464K | I175A | G2665S | M2666W | L2694Y |
| Mycobacterium tuberculosis | H8EU44 | 49.9% | 54.6% | 34.4% | 61.8% | R1464K | I175A | G2665S | M2666W | L2694Y |
| | | | | | | | | | | |
| Tsukamurella paurometabola | D5UWS2 | 49.4% | 52.1% | 37.3% | 64.4% | R1490K | I198A | G2686S | M2687W | L2715Y |
| | | | | | | | | | | |
| Actinomyces odontolyticus | A7BDJ6 | 47.3% | 55.7% | 33.6% | 58.3% | R1442K | I156A | G2657S | F2658W | L2686Y |
| Bifidobacterium adolescentis | A1A004 | 46.2% | 55.4% | 33.0% | 60.9% | 1467K | I158A | G2703S | F2704W | L2732Y |

**Table 2: Fatty acid synthases similar to the Corynebacterium ammoniagenes FAS.**

| The mutations that were used to install a system of chain length control, can easily be applied to other fatty acid synthases as well. Here, an overview of suitable FAS systems is listed with their sequence identity to whole type I FAS chain of the *C*. *ammoniagenes* FAS as they are listed in Uniprot. | | |
|---|---|---|
| *UniProt identifier* | *Organism ID* | *Sequence Identity with C. ammoniagenes FAS (D5NXL2)* |
| D5NXL2 | Corynebacterium ammoniagenes DSM 20306 | |
| Q04846 | Corynebacterium ammoniagenes (Brevibacterium ammoniagenes) | Ident.: 99.7% |
| G7HWL7 | Corynebacterium casei UCMA 3821 | Ident.: 87.9% |
| W5XVW4 | Corynebacterium casei LMG S-19264 | Ident.: 87.9% |
| C2CLH5 | Corynebacterium striatum ATCC 6940 | Ident.: 68.2% |
| A0A0B5DCR4 | Corynebacterium humireducens NBRC 106098 = DSM 45392 | Ident.: 65.1% |
| A0A0B6TI31 | Corynebacterium marinum DSM 44953 | Ident.: 64.3% |
| M1NPI7 | Corynebacterium halotolerans YIM 70093 = DSM 44683 | Ident.: 63.8% |
| W5Y2K5 | Corynebacterium vitaeruminis DSM 20294 | Ident.: 63.4% |
| Q8FMV7 | Corynebacterium efficiens (strain DSM 44549 / YS-314 / AJ 12310 / JCM 11189 / NBRC 100395) | Ident.: 63.1% |
| Q8NMS0 | Corynebacterium glutamicum (strain ATCC 13032 / DSM 20300 / JCM 1318 / LMG 3730 /NCIMB 10025) | Ident.: 62.4% |
| Q6M2X6 | Corynebacterium glutamicum (strain ATCC 13032 / DSM 20300 / JCM 1318 / LMG 3730 / NCIMB 10025) | Ident.: 62.4% |
| I0LMA2 | Corynebacterium glutamicum (strain ATCC 13032/K051) | Ident.: 62.4% |
| A4QGP8 | Corynebacterium glutamicum (strain R) | Ident.: 62.2% |
| M1V022 | Corynebacterium callunae DSM 20147 | Ident.: 62.4% |
| R0HZN0 | Corynebacterium crenatum MT | Ident.: 62.1 % |
| AOA072ZAJ1 | Corynebacterium glutamicum ATCC 14067 | Ident.: 62.0% |
| L1MJZ0 | Corynebacterium durum F0235 | Ident.: 61.5% |
| S5SWZ0 | Corynebacterium maris DSM 45190 | Ident.: 61.7% |
| G0CX25 | Corynebacterium ulcerans (strain BR-AD22) | Ident.: 61.1% |
| V6V555 | Corynebacterium ulcerans NCTC 12077 | Ident.: 60.9% |
| D9QC32 | Corynebacterium pseudotuberculosis (strain C231) | Ident.: 61.0% |
| D8KPC3 | Corynebacterium pseudotuberculosis (strain FRC41) | Ident.: 61.0% |
| D9Q461 | Corynebacterium pseudotuberculosis (strain 1002) | Ident.: 61.0% |
| A0A0A8BAK1 | Corynebacterium pseudotuberculosis | Ident.: 61.0% |
| C0E3S2 | Corynebacterium matruchotii ATCC 33806 | Ident.: 61.0% |
| E0DGU8 | Corynebacterium matruchotii ATCC 14266 | Ident.: 61.1% |
| H2I0C4 | Corynebacterium diphtheriae (strain PW8) | Ident.: 61.4% |
| Q6NFP1 | Corynebacterium diphtheriae (strain ATCC 700971 / NCTC 13129 / Biotype gravis) | Ident.: 61.4% |
| H2GWD3 | Corynebacterium diphtheriae (strain ATCC 27012 / C7 (beta)) | Ident.: 61.3% |
| C3PIL8 | Corynebacterium aurimucosum (strain ATCC 700975 / DSM 44827 / CN-1) (Corynebacterium nigricans) | Ident.: 60.5% |
| A0A0B2YX13 | Corynebacterium minutissimum | Ident.: 60.8% |
| A0A0B6F6M2 | Corynebacterium singulare | Ident.: 59.8% |
| U3GVJ3 | Corynebacterium argentoratense DSM 44202 | Ident.: 59.8% |
| AOA095Y8K9 | Corynebacterium freneyi DNF00450 | Ident.: 56.1% |
| S2XC88 | Corynebacterium sp. HFH0082 | Ident.: 54.6% |
| E2MXG2 | Corynebacterium amycolatum SK46 | Ident.: 54.6% |
| A0A088QGW3 | Corynebacterium sp. ATCC 6931 | Ident.: 54.3% |
| A0A059MRB8 | Rhodococcus sp. BCP1 | Ident.: 52.6% |
| N1M581 | Rhodococcus sp. EsD8 | Ident.: 52.6% |
| W4A6R5 | Rhodococcus rhodochrous ATCC 21198 | Ident.: 52.5% |
| AOA098BIF 1 | Rhodococcus ruber | Ident.: 52.3% |
| M2YCX3 | Rhodococcus ruber BKS 20-38 | Ident.: 52.2% |
| Q5Z0D9 | Nocardia farcinica (strain IFM 10152) | Ident.: 51.8% |
| AOA022LQ86 | Dietzia sp. UCD-THP | Ident.: 51.7% |
| H0JYN0 | Rhodococcus pyridinivorans AK37 | Ident.: 51.7% |
| V9XB62 | Rhodococcus pyridinivorans SB3094 | Ident.: 51.7% |
| E4WJI2 | Rhodococcus equi (strain 103S) (Corynebacterium equi) | Ident.: 51.4% |
| X0Q921 | Rhodococcus wratislaviensis NBRC 100605 | Ident.: 51.4% |
| C1AVV2 | Rhodococcus opacus (strain B4) | Ident.: 51.4% |
| A0A076EUA2 | Rhodococcus opacus (Nocardia opaca) | Ident.: 51.4% |
| E9T4J9 | Rhodococcus equi ATCC 33707 | Ident.: 51.3% |
| I0WW98 | Rhodococcus imtechensis RKJ300 = JCM 13270 | Ident.: 51.5% |
| A0A0B1RFX0 | Rhodococcus sp. Chr-9 | Ident.: 51.7% |
| K8XCY2 | Rhodococcus opacus M213 | Ident.: 51.5% |
| L8DG25 | Rhodococcus sp. AW25M09 | Ident.: 51.8% |
| H6R4J7 | Nocardia cyriacigeorgica (strain GUH-2) | Ident.: 51.1% |
| M2XKX3 | Rhodococcus triatomae BKS 15-14 | Ident.: 51.7% |
| J2JKR0 | Rhodococcus sp. JVH1 | Ident.: 51.3% |
| Q0SGU1 | Rhodococcus jostii (strain RHA1) | Ident.: 51.3% |
| K0EVA9 | Nocardia brasiliensis ATCC 700358 | Ident.: 51.2% |
| C3JU60 | Rhodococcus erythropolis SK121 | Ident.: 51.4% |
| M2V5E8 | Rhodococcus qingshengii BKS 20-40 | Ident.: 51.4% |
| U5EKQ0 | Nocardia asteroides NBRC 15531 | Ident.: 51.5% |
| A0A0D0L8N1 | Rhodococcus sp. MEB064 | Ident.: 51.4% |
| A0A034UN97 | Nocardia brasiliensis NBRC 14402 | Ident.: 51.2% |
| T1VRH4 | Rhodococcus erythropolis CCM2595 | Ident.: 51.4% |
| C1A1U6 | Rhodococcus erythropolis (strain PR4 / NBRC 100887) | Ident.: 51.4% |
| A0A0A1FSM8 | Mycobacterium sp. VKM Ac-1817D | Ident.: 51.1% |
| K0VSI7 | Mycobacterium fortuitum subsp. fortuitum DSM 46621 | Ident.: 51.1% |
| A0A024M7D7 | Mycobacterium farcinogenes | Ident.: 50.9% |
| A0A0B8NBN9 | Nocardia seriolae | Ident.: 50.7% |
| R7WMD2 | Rhodococcus rhodnii LMG 5362 | Ident.: 51.4% |
| F6ES54 | Amycolicicoccus subflavus (strain DSM 45089 / DQS3-9A1) | Ident.: 51.4% |
| A0A0B2YJI1 | Mycobacterium setense | Ident.: 51.0% |
| A0A0B2Y2N4 | Mycobacterium setense | Ident.: 50.9% |
| X5M0C1 | Mycobacterium vulneris | Ident.: 50.9% |
| A0R1H7 | Mycobacterium smegmatis (strain ATCC 700084 / mc(2)155) | Ident.: 50.8% |
| I0RH15 | Mycobacterium phlei RIVM601174 | Ident.: 50.7% |
| V5XFS4 | Mycobacterium neoaurum VKM Ac-1815D | Ident.: 50.7% |
| A0A024QWT5 | Mycobacterium neoaurum | Ident.: 50.6% |
| H6MX15 | Gordonia polyisoprenivorans (strain DSM 44266 / VH2) | Ident.: 51.1% |
| X5LF08 | Mycobacterium mageritense | Ident.: 50.8% |
| W9AU18 | Mycobacterium cosmeticum | Ident.: 50.3% |
| F5YXB4 | Mycobacterium sp. (strain JDM601) | Ident.: 50.7% |
| H5UAN8 | Gordonia terrae NBRC 100016 | Ident.: 50.8% |
| L7KCY3 | Gordonia rubripertincta NBRC 101908 | Ident.: 50.8% |
| M0QLC3 | Gordonia soli NBRC 108243 | Ident.: 51.0% |
| L7LK57 | Gordonia sihwensis NBRC 108236 | Ident.: 51.1% |
| K0V149 | Mycobacterium vaccae ATCC 25954 | Ident.: 50.9% |
| Q6XXM0 | Mycobacterium smegmatis | Ident.: 50.6% |
| G7CJQ0 | Mycobacterium thermoresistibile ATCC 19527 | Ident.: 50.4% |
| L0J007 | Mycobacterium smegmatis JS623 | Ident.: 50.2% |
| I4BLY3 | Mycobacterium chubuense (strain NBB4) | Ident.: 50.8% |
| Q1B5S9 | Mycobacterium sp. (strain MCS) | Ident.: 50.5% |
| A1UJA7 | Mycobacterium sp. (strain KMS) | Ident.: 50.5% |
| M3URF9 | Gordonia paraffinivorans NBRC 108238 | Ident.: 50.3% |
| K6XSY7 | Gordonia namibiensis NBRC 108229 | Ident.: 50.4% |
| A0A099CNA1 | Mycobacterium rufum | Ident.: 50.9% |
| L7L493 | Gordonia amicalis NBRC 100051 = JCM 11271 | Ident.: 50.5% |
| I0RQT2 | Mycobacterium xenopi RIVM700367 | Ident.: 50.3% |
| D0LAD4 | Gordonia bronchialis (strain ATCC 25592 / DSM 43247 / JCM 3198 / NCTC 10667) (Rhodococcus bronchialis) | Ident.: 50.8% |
| A3Q2R1 | Mycobacterium sp. (strain JLS) | Ident.: 50.3% |
| R7YD01 | Gordonia terrae C-6 | Ident.: 50.5% |
| J9SDV9 | Gordonia sp. KTR9 | Ident.: 50.5% |
| K5BEE6 | Mycobacterium hassiacum DSM 44199 | Ident.: 49.8% |
| H8IPF6 | Mycobacterium intracellulare (strain ATCC 13950 / DSM 43223 / JCM 6384 / NCTC 13025 / 3600) | Ident.: 50.3% |
| Q73XH7 | Mycobacterium paratuberculosis (strain ATCC BAA-968 / K-10) | Ident.: 50.3% |
| W9DK11 | Gordonia alkanivorans CGMCC 6845 | Ident.: 50.4% |
| F9VRD7 | Gordonia alkanivorans NBRC 16433 | Ident.: 50.4% |
| L7L921 | Gordonia hirsuta DSM 44140 = NBRC 16056 | Ident.: 50.8% |
| X7UCI3 | Mycobacterium avium MAV_120709_2344 | Ident.: 50.3% |
| T2GPR8 | Mycobacterium avium subsp. hominissuis TH135 | Ident.: 50.3% |
| V7JLG4 | Mycobacterium avium 10-5581 | Ident.: 50.3% |
| A0QD96 | Mycobacterium avium (strain 104) | Ident.: 50.3% |
| X7VP42 | Mycobacterium sp. MAC_080597_8934 | Ident.: 50.3% |
| V7J6Y6 | Mycobacterium avium 05-4293 | Ident.: 50.3% |
| AOA049E2Y8 | Mycobacterium avium XTB13-223 | Ident.: 50.3% |
| A0A081HXZ8 | Mycobacterium sp. TKK-01-0059 | Ident.: 50.3% |
| F1YQ19 | Gordonia neofelifaecis NRRL B-59395 | Ident.: 51.0% |
| A4T7Z1 | Mycobacterium gilvum (strain PYR-GCK) (Mycobacterium flavescens (strain ATCC 700033 / PYR-GCK)) | Ident.: 50.7% |
| I2AAV6 | Mycobacterium sp. MOTT36Y | Ident.: 50.3% |
| G7GMR0 | Gordonia amarae NBRC 15530 | Ident.: 50.5% |
| J9WBT0 | Mycobacterium indicus pranii MTCC 9506 | Ident.: 50.3% |
| A1TCK2 | Mycobacterium vanbaalenii (strain DSM 7251 / PYR-1) | Ident.: 50.5% |
| E6TCW8 | Mycobacterium sp. (strain Spyr1) | Ident.: 50.7% |
| A0A064CM15 | Mycobacterium aromaticivorans JS19b1 = JCM 16368 | Ident.: 50.4% |
| X8A5U7 | Mycobacterium avium subsp. avium 2285 (S) | Ident.: 50.3% |
| A0A0D1LRC7 | Mycobacterium llatzerense | Ident.: 49.7% |
| H5U4Q5 | Gordonia sputi NBRC 100414 | Ident.: 50.3% |
| G4I1Q2 | Mycobacterium rhodesiae JS60 | Ident.: 50.0% |
| X7SGD2 | Mycobacterium intracellulare MIN_052511_1280 | Ident.: 50.4% |
| S4Z6F0 | Mycobacterium yongonense 05-1390 | Ident.: 50.3% |
| L7KR89 | Gordonia aichiensis NBRC 108223 | Ident.: 50.1% |
| D5PGZ3 | Mycobacterium parascrofulaceum ATCC BAA-614 | Ident.: 50.2% |
| | | |
| M3UKD4 | Gordonia malaquae NBRC 108250 | Ident.: 50.6% |
| G8RH96 | Mycobacterium rhodesiae (strain NBB3) | Ident.: 49.4% |
| U5WKV0 | Mycobacterium kansasii ATCC 12478 | Ident.: 50.1% |
| A0A0D1L7L0 | Mycobacterium immunogenum | Ident.: 50.0% |
| A1KLM0 | Mycobacterium bovis (strain BCG / Pasteur 1173P2) | Ident.: 49.9% |
| Q7TYD8 | Mycobacterium bovis (strain ATCC BAA-935 / AF2122/97) | Ident.: 49.9% |
| C1AEZ1 | Mycobacterium bovis (strain BCG / Tokyo 172 / ATCC 35737 / TMC 1019) | Ident.: 49.9% |
| A0A056FVK2 | Mycobacterium tuberculosis BTB05-348 | Ident.: 49.9% |
| F9UUW4 | Mycobacterium bovis BCG str. Moreau RDJ | Ident.: 49.9% |
| A0A083W2V7 | Mycobacterium bovis | Ident.: 49.9% |
| A0A045I7N8 | Mycobacterium tuberculosis | Ident.: 49.9% |
| GOTQ02 | Mycobacterium canettii (strain CIPT 140010059) | Ident.: 49.8% |
| M8CK50 | Mycobacterium orygis 112400015 | Ident.: 49.8% |
| A0A051TX33 | Mycobacterium tuberculosis TKK-01-0051 | Ident.: 49.9% |
| A0A0C7DCE1 | Mycobacterium bovis | Ident.: 49.9% |
| C6DMH3 | Mycobacterium tuberculosis (strain KZN 1435 / MDR) | Ident.: 49.9% |
| A5WQD3 | Mycobacterium tuberculosis (strain F11) | Ident.: 49.9% |
| H8EU44 | Mycobacterium tuberculosis (strain ATCC 35801 / TMC 107 / Erdman) | Ident.: 49.9% |
| A5U5M2 | Mycobacterium tuberculosis (strain ATCC 25177 / H37Ra) | Ident.: 49.9% |
| P95029 | Mycobacterium tuberculosis (strain ATCC 25618 / H37Rv) | Ident.: 49.9% |
| AOA056EYB5 | Mycobacterium tuberculosis BTB05-013 | Ident.: 49.9% |
| A0A047RTH0 | Mycobacterium tuberculosis OFXR-28 | Ident.: 49.9% |
| A0A040D5G9 | Mycobacterium tuberculosis MD15956 | Ident.: 49.9% |
| F8M2M4 | Mycobacterium africanum (strain GM041182) | Ident.: 49.8% |
| J5E4H3 | Mycobacterium colombiense CECT 3035 | Ident.: 49.7% |
| Q7D6Z3 | Mycobacterium tuberculosis (strain CDC 1551 / Oshkosh) | Ident.: 49.8% |
| D6FJ88 | Mycobacterium tuberculosis CPHL_A | Ident.: 49.8% |
| B2HPL8 | Mycobacterium marinum (strain ATCC BAA-535 / M) | Ident.: 49.4% |
| A0A097U833 | Mycobacterium abscessus | Ident.: 49.7% |
| I0PEP9 | Mycobacterium abscessus M94 | Ident.: 49.7% |
| B1MMN6 | Mycobacterium abscessus (strain ATCC 19977 / DSM 44196) | Ident.: 49.6% |
| A0A097TL16 | Mycobacterium abscessus subsp. bolletii | Ident.: 49.6% |
| X8EBI6 | Mycobacterium chelonae 1518 | Ident.: 49.6% |
| H0IMG8 | Mycobacterium abscessus subsp. bolletii BD | Ident.: 49.7% |
| A0A024K1L7 | Mycobacterium triplex | Ident.: 49.2% |
| R4U8X5 | Mycobacterium abscessus subsp. bolletii 50594 | Ident.: 49.6% |
| A0A089UN39 | Mycobacterium abscessus subsp. bolletii | Ident.: 49.6% |
| L7VAX9 | Mycobacterium liflandii (strain 128FXT) | Ident.: 49.1% |
| G7H181 | Gordonia araii NBRC 100433 | Ident.: 50.3% |
| A0PU94 | Mycobacterium ulcerans (strain Agy99) | Ident.: 49.1% |
| H0R6C2 | Gordonia effusa NBRC 100432 | Ident.: 49.7% |
| E2SBC7 | Aeromicrobium marinum DSM 15272 | Ident.: 50.1% |
| D5UWS2 | Tsukamurella paurometabola (strain ATCC 8368 / DSM 20162 / JCM 10117 / NBRC 16120 / NCTC 13040) (Corynebacterium paurometabolum) | Ident.: 49.4% |
| B8ZR71 | Mycobacterium leprae (strain Br4923) | Ident.: 49.0% |
| Q7AQ85 | Mycobacterium leprae (strain TN) | Ident.: 49.0% |
| Q9X7E2 | Mycobacterium leprae | Ident.: 49.0% |
| W8H3V7 | Rhodococcus opacus PD630 | Ident.: 53.7% |
| U1N9D8 | Segniliparus rugosus ATCC BAA-974 | Ident.: 48.6% |
| F2UZ57 | Actinomyces viscosus C505 | Ident.: 48.1% |
| F9PKDO | Actinomyces sp. oral taxon 175 str. F0384 | Ident.: 48.0% |
| D6ZBM5 | Segniliparus rotundus (strain ATCC BAA-972 / CDC 1076 / CIP 108378 / DSM 44985 / JCM 13578) | Ident.: 47.5% |
| F3PAT7 | Actinomyces sp. oral taxon 170 str. F0386 | Ident.: 48.0% |
| S2ZRS9 | Actinomyces sp. HPA0247 | Ident.: 47.5% |
| F9EGT4 | Actinomyces sp. oral taxon 448 str. F0400 | Ident.: 48.2% |
| X5DQL0 | Corynebacterium glycinophilum AJ 3170 | Ident.: 47.2% |
| Z4XC48 | Actinomyces sp. ICM54 | Ident.: 47.2% |
| A7BDJ6 | Actinomyces odontolyticus ATCC 17982 | Ident. : 47.3% |
| E6KQH3 | Actinomyces sp. oral taxon 180 str. F0310 | Ident.: 47.4% |
| J1GZW8 | Actinomyces massiliensis F0489 | Ident.: 47.8% |
| G9PE60 | Actinomyces graevenitzii C83 | Ident.: 46.9% |
| G0HFH2 | Corynebacterium variabile (strain DSM 44702 / JCM 12073 / NCIMB 30131) (Corynebacterium mooreparkense) | Ident.: 46.9% |
| S4XF69 | Corynebacterium terpenotabidum Y-11 1 | Ident.: 46.4% |
| D2Q8A3 | Bifidobacterium dentium (strain ATCC 27534 / DSM 20436 / JCM 1195 / Bdl) | Ident.: 46.1% |
| A0A0C6E7J8 | Bifidobacterium dentium JCM 1195 = DSM 20436 | Ident.: 46.1% |
| A0A076JKG5 | Bifidobacterium adolescentis | Ident.: 46.2% |
| A0A087DLN6 | Bifidobacterium stercoris | Ident.: 46.2% |
| E0Q8W2 | Bifidobacterium dentium ATCC 27679 | Ident.: 46.3% |
| W2VJ86 | Bifidobacterium sp. MSTE12 | Ident.: 46.1% |
| A0A0A7I0W6 | Bifidobacterium kashiwanohense PV20-2 | Ident.: 46.5% |
| A0A0B5BMG2 | Bifidobacterium adolescentis | Ident.: 46.1% |
| A7A3U5 | Bifidobacterium adolescentis L2-32 | Ident.: 46.2% |
| A1A004 | Bifidobacterium adolescentis (strain ATCC 15703 / DSM 20083 / NCTC 11814/E194a) | Ident.: 46.2% |
| A0A072MVF6 | Bifidobacterium pseudocatenulatum IPLA36007 | Ident.: 46.5% |
| S2VFA7 | Actinobaculum schaalii FB123-CNA-2 | Ident.: 47.2% |
| W4N6R1 | Bifidobacterium moukalabense DSM 27321 | Ident.: 46.2% |
| A0A068NFS0 | Actinobaculum schaalii | Ident.: 47.2% |
| A0A0C2YRD0 | Bifidobacterium adolescentis | Ident.: 46.1% |
| C0BQR0 | Bifidobacterium pseudocatenulatum DSM 20438 = JCM 1200 = LMG 10505 | Ident.: 46.4% |
| A0A087AXX9 | Bifidobacterium kashiwanohense JCM 15439 = DSM 21854 | Ident.: 46.4% |
| B6XWG4 | Bifidobacterium catenulatum DSM 16992 = JCM 1194 = LMG 11043 | Ident.: 46.5% |
| D1NVL5 | Bifidobacterium gallicum DSM 20093 = LMG 11596 | Ident.: 46.1% |
| A0A095X260 | Actinomyces urogenitalis S6-C4 | Ident.: 46.7% |
| AOA087CW62 | Bifidobacterium ruminantium | Ident.: 46.1% |
| L1MKX4 | Corynebacterium durum F0235 | Ident.: 46.6% |
| H0RHZ8 | Gordonia polyisoprenivorans NBRC 16320 = JCM 10675 | Ident.: 52.7% |
| W4HVU8 | Mycobacterium gastri 'Wayne' | Indent. 51.2% |
| U1QQP8 | Alloscardovia omnicolens F0580 | Ident.: 45.4% |
| A0A087CFP3 | Bifidobacterium psychraerophilum | Ident.: 45.2% |
| A0A0C6DV43 | Bifidobacterium scardovii JCM 12489 = DSM 13734 | Ident.: 45.5% |
| A0A087D458 | Bifidobacterium scardovii | Ident.: 45.5% |
| X4QU22 | Trueperella pyogenes | Ident.: 46.1% |
| A0A0A8FKW3 | Trueperella pyogenes TP8 | Ident.: 46.1% |
| E6K0M0 | Parascardovia denticolens DSM 10105 = JCM 12538 | Ident.: 45.2% |
| D0WNR5 | Actinomyces sp. oral taxon 848 str. F0332 | Ident.: 46.4% |
| L1PM94 | Actinomyces sp. oral taxon 181 str. F0379 | Ident.: 45.4% |
| D4BQH0 | Bifidobacterium breve DSM 20213 = JCM 1192 | Ident.: 45.3% |
| F6C810 | Bifidobacterium breve (strain ACS-071-V-Sch8b) | Ident.: 45.3% |
| AOA087E619 | Bifidobacterium thermacidophilum subsp. thermacidophilum | Ident.: 45.0% |
| A0A0A2DGH8 | Corynebacterium auriscanis | Ident.: 46.3% |
| F9XYS6 | Bifidobacterium breve (strain NCIMB 8807 / UCC2003) | Ident.: 45.4% |
| E4VBF4 | Bifidobacterium bifidum NCIMB 41171 | Ident.: 45.7% |
| A0A080N4A6 | Bifidobacterium bombi DSM 19703 | Ident.: 45.1% |
| W6F806 | Bifidobacterium breve NCFB 2258 | Ident.: 45.2% |
| E3EN51 | Bifidobacterium bifidum (strain S17) | Ident.: 45.7% |
| M4RE52 | Bifidobacterium thermophilum RBL67 | Ident.: 45.1% |
| I8UNY7 | Parascardovia denticolens IPLA 20019 | Ident.: 45.1% |
| S2ZNV1 | Bifidobacterium breve HPH0326 | Ident.: 45.2% |
| N6W6S0 | Actinomyces cardiffensis F0333 | Ident.: 45.5% |
| A0A095YXL1 | Actinomyces sp. S6-Spd3 | Ident.: 45.4% |
| I3WJZ1 | Bifidobacterium bifidum BGN4 | Ident.: 45.7% |
| I1W8H0 | Bifidobacterium animalis subsp. animalis (strain ATCC 25527 / DSM 20104 / JCM 1190 / R101-8) | Ident.: 45.4% |
| A0A086YYH8 | Bifidobacterium actinocoloniiforme DSM 22766 | Ident.: 44.9% |
| A0AOB6VZA0 | Bifidobacterium animalis subsp. animalis IM386 | Ident.: 45.4% |
| K9F0D2 | Actinobaculum massiliae ACS-171-V-Col2 | Ident.: 46.0% |
| AOA087E7Z7 | Bifidobacterium subtile | Ident.: 45.1 % |

The polypeptides of the present invention, in particular the type I FAS variants, have an *in vitro* and/or *in vivo* fatty acid synthase I (FAS I) enzymatic activity.

As used herein, the term "percent (%) identical" refers to sequence identity between two amino acid sequences. Identity can be determined by comparing a position in both sequences, which may be aligned for the purpose of comparison. When an equivalent position in the compared sequences is occupied by the same amino acid, the molecules are considered to be identical at that position.

As used herein, the term "functional equivalent" refers to amino acid sequences that are not 100% identical to the amino acid sequence of SEQ ID NO. 1, 2 or 3 and comprise amino acid additions and/or insertions and/or deletions and/or substitutions and/or exchanges, which do not alter or change the activity or function of the protein as compared to the protein having the amino acid sequence of SEQ ID NO: 1, 2 or 3, i.e. an "functional equivalent", for example, encompasses an amino acid sequence with conservative amino acid substitutions or smaller deletions and/or insertions as long as these modifications do not substantially affect the *in vitro* and/or *in vivo* fatty acid synthase (FAS) enzymatic activity.

Generally, a person skilled in the art is aware of the fact that some amino acid exchanges in the amino acid sequence of a protein do not have an influence on the (secondary or tertiary) structure, function and/or activity of that protein. Amino acid sequences with such "neutral" amino acid exchanges as compared to the amino acid sequences disclosed herein fall within the scope of the present invention.

In some embodiments, the polypeptide(s) or protein(s) have one, two, three, four, five, six or more amino acid substitutions in the MPT domain, AT domain and/or KS domain.

For example, a polypeptide of the present inventions has one amino acid substitution in each of the MPT domain, AT domain and/or KS domain.

For example, a polypeptide of the present inventions has two amino acid substitutions, such as:
one amino acid substitution in each of the MPT domain and the AT domain,
one amino acid substitution in each of the MPT domain and the KS domain,
one amino acid substitution in each of the AT domain and the KS domain,
two amino acid substitutions in the MPT domain,
two amino acid substitutions in the AT domain,
two amino acid substitutions in the KS domain.

For example, a polypeptide of the present inventions has three amino acid substitutions, such as:
three amino acid substitutions in the MPT domain,
three amino acid substitutions in the AT domain,
three amino acid substitutions in the KS domain.
two amino acid substitutions in the MPT domain and one amino acid substitution in either one of the AT domain and the KS domain,
two amino acid substitutions in the AT domain and one amino acid substitution in either one of the MPT domain and the KS domain,
two amino acid substitutions in the KS domain and one amino acid substitution in either one of MPT domain and the AT domain,
one amino acid substitution in each of the MPT domain, AT domain and KS domain.

Preferably, the polypeptides or proteins according to the present invention comprise
- the amino acid substitution R90K in the MPT domain (SEQ ID NO. 1), and/or
- the amino acid substitution I151A in the AT domain (SEQ ID NO: 2).

Preferably, the polypeptides or proteins according to the present invention comprise amino acid substitution(s) in the acyl binding channel of the KS domain, more preferably amino acid substitution(s) G227S, M228W and/or L256Y in the KS domain (SEQ ID NO: 3).

Preferably, the polypeptides or proteins according to the present invention comprise
- the amino acid substitution R90K in the MPT domain (SEQ ID NO. 1),
   and/or
- the amino acid substitution I151A in the AT domain (SEQ ID NO: 2).
   and/or
the amino acid substitution(s) G227S, M228W and/or L256Y in the KS domain (SEQ ID NO: 3).

Preferably, the proteins or polypeptides according to the present invention are selected from the group of
- a protein comprising the amino acid substitution G227S;
- a protein comprising the amino acid substitutions G227S and M228W;
- a protein comprising the amino acid substitutions I151A, G227S and M228W;
- a protein comprising the amino acid substitutions R90K, G227S and M228W; and
- a protein comprising the amino acid substitutions I151A, R90K, G227S and M228W;
wherein
R90K refers to R90K in the MPT domain of the amino acid sequence of SEQ ID NO: 1; I151A refers to I151A in the AT domain of the amino acid sequence of SEQ ID NO: 2; G227S refers to G227S in the KS domain of the amino acid sequence of SEQ ID NO: 3; M228W refers to M228W in the KS domain of the amino acid sequence of SEQ ID NO: 3; and L256Y refers to L256Y in the KS domain of the amino acid sequence of SEQ ID NO: 3.

Preferably, the proteins or polypeptides according to the present invention are selected from the group of
- a protein comprising the amino acid substitution G227S;
- a protein comprising the amino acid substitutions I151A and G227S;
- a protein comprising the amino acid substitutions I151A, R90K and L256Y;
- a protein comprising the amino acid substitutions I151A, R90K and G227S;
- a protein comprising the amino acid substitution R90K;
- a protein comprising the amino acid substitutions I151A, R90K, G227S and M228W;
- a protein comprising the amino acid substitutions I151A, G227S and M228W;
- a protein comprising the amino acid substitutions G227S and M228W;
- a protein comprising the amino acid substitutions I151A and R90K;
- a protein comprising the amino acid substitutions R90K and G227S;
- a protein comprising the amino acid substitutions I151A and L256Y;
- a protein comprising the amino acid substitutions I151A, G227S and L256Y;
- a protein comprising the amino acid substitutions R90K, G227S and M228W;
- a protein comprising the amino acid substitutions G227S and L256Y;
- a protein comprising the amino acid substitutions R90K, G227S and L256Y;
- a protein comprising the amino acid substitutions I151A, R90K, G227S and L256Y;
- a protein comprising the amino acid substitutions I151A, G227S, M228W and L256Y;
- a protein comprising the amino acid substitution I151A;
- a protein comprising the amino acid substitution M228W;
- a protein comprising the amino acid substitution L256Y;
- a protein comprising the amino acid substitutions I151A and M228W;
- a protein comprising the amino acid substitutions R90K and M228W;
- a protein comprising the amino acid substitutions R90K and L256Y;
- a protein comprising the amino acid substitutions I151A, R90K and M228W;
- a protein comprising the amino acid substitutions I151A, M228W and L256Y;
- a protein comprising the amino acid substitutions R90K, M228W and L256Y;
- a protein comprising the amino acid substitutions G227S, M228W and L256Y;
- a protein comprising the amino acid substitutions M228W and L256Y;
- a protein comprising the amino acid substitutions I151A, R90K, M228W and L256Y;
- a protein comprising the amino acid substitutions R90K, G227S, M228W and L256Y; and
- a protein comprising the amino acid substitutions I151A, R90K, G227S, M228W and L256Y,
wherein
R90K refers to R90K in the MPT domain of the amino acid sequence of SEQ ID NO: 1; I151A refers to I151A in the AT domain of the amino acid sequence of SEQ ID NO: 2; G227S refers to G227S in the KS domain of the amino acid sequence of SEQ ID NO: 3; M228W refers to M228W in the KS domain of the amino acid sequence of SEQ ID NO: 3; and L256Y refers to L256Y in the KS domain of the amino acid sequence of SEQ ID NO: 3.

The present invention preferably provides the following proteins or polypeptides / type I FAS variants:
variant G227S;
variant G227S / M228W;
variant I151A / G227S / M228W;
variant R90K / G227S / M228W;
variant I151A / R90K / G227S / M228W;
wherein
R90K refers to R90K in the MPT domain of the amino acid sequence of SEQ ID NO: 1; I151A refers to I151A in the AT domain of the amino acid sequence of SEQ ID NO: 2; G227S refers to G227S in the KS domain of the amino acid sequence of SEQ ID NO: 3; and M228W refers to M228W in the KS domain of the amino acid sequence of SEQ ID NO: 3.

The present invention preferably provides the following proteins or polypeptides / type I FAS variants:
variant G227S;
variant I151A / G227S;
variant I151A / R90K / L256Y;
variant I151A / R90K / G227S;
variant R90K;
variant I151A / R90K / G227S / M228W;
variant I151A / G227S / M228W;
variant G227S / M228W;
variant I151A / R90K;
variant R90K / G227S;
variant I151A / L256Y;
variant I151A / G227S / L256Y;
variant R90K / G227S / M228W;
variant G227S / L256Y;
variant R90K / G227S / L256Y;
variant I151A / R90K / G227S / L256Y;
variant I151A / G227S / M228W / L256Y;
variant I151A;
variant M228W;
variant L256Y;
variant I151A / M228W;
variant R90K / M228W;
variant R90K / L256Y;
variant I151A / R90K / M228W;
variant I151A / M228W / L256Y;
variant R90K / M228W / L256Y;
variant G227S / M228W / L256Y;
variant M228W / L256Y;
variant I151A / R90K / M228W / L256Y;
variant R90K / G227S / M228W / L256Y; and
variant I151A / R90K / G227S / M228W / L256Y,
wherein
R90K refers to R90K in the MPT domain of the amino acid sequence of SEQ ID NO: 1; I151A refers to I151A in the AT domain of the amino acid sequence of SEQ ID NO: 2; G227S refers to G227S in the KS domain of the amino acid sequence of SEQ ID NO: 3; M228W refers to M228W in the KS domain of the amino acid sequence of SEQ ID NO: 3; and L256Y refers to L256Y in the KS domain of the amino acid sequence of SEQ ID NO: 3.

Preferably, the protein (s) or polypeptide(s) of the present invention result in elevated overall production of short fatty acids, CoA esters of short fatty acids, ethyl esters of short fatty acids, esters of short fatty acids with other metabolites, and/or enzyme bound short fatty acids (C₆ to C₁₂) compared to the wild type polypeptide(s) or the polypeptide(s) without such amino acid substitution(s).
The elevated overall production of short fatty acids, CoA esters of short fatty acids, short fatty acid ethyl esters, short fatty acid esters with other metabolites, and/or enzyme bound short fatty acids (C₆ to C₁₂) is increased at least 2-fold, or preferably at least 5-fold or 10-fold or 20-fold or 27-fold.

The term "short fatty acid" refers to a fatty acid of short to medium length with C₆ to C₁₂ in their free form or bound to CoA molecule (the typical form in which they are unloaded from the enzyme).

In one embodiment, the polypeptide(s) of the present invention show(s) an increased selectivity for the production of C₆ fatty acids, C₆ CoA esters, C₆ fatty acid ethyl esters, C₆ fatty acid esters with other metabolites, and/or enzyme bound C₆ fatty acids compared to wild type polypeptide(s) or the polypeptide without such amino acid substitution(s).

The selectivity for the production of C₆ fatty acids, C₆ CoA esters, C₆ fatty acid ethyl esters, C₆ fatty acid esters with other metabolites, and/or enzyme bound C₆ fatty acids compared to wild type is increased where the share of C₆ makes up at least 30%, or preferably at least 40% or 50% or 70% or 80% or 90% of the detected chain length between C₆ and C₁₂.

In one embodiment, the protein (s) or polypeptide(s) of the present invention show(s) an increased selectivity for the production of C₈ fatty acids, C₈ fatty acid CoA esters, C₈ fatty acid ethyl esters, C₈ fatty acid esters with other metabolites, and/or enzyme bound C₈ fatty acids compared to wild type polypeptide(s) or the polypeptide without such amino acid substitution(s).

The selectivity for the production of C₈ fatty acids, C₈ fatty acid CoA esters and/or enzyme bound C₈ fatty acids compared to wild type is increased where the share of Cg makes up at least 30%, or preferably at least 40% or 50% or 70% or 80% or 89% of the detected chain length between C₆ and Cₗ₂.

In one embodiment, the protein (s) or polypeptide(s) of the present invention show(s) an increased selectivity for the production of C₁₀ to C₁₂ fatty acids, C₁₀ to C₁₂ fatty acid CoA esters, C₁₀ to C₁₂ fatty acid ethyl esters, C₁₀ to C₁₂ fatty acid esters with other metabolites, and/or enzyme bound C₁₀ to C₁₂ fatty acids compared to wild type polypeptide(s) or the polypeptide without such amino acid substitution(s).

For example, proteins or polypeptides of the present invention, wherein the amino acid substitution(s) is/are selected from
1151A (in the AT domain on SEQ ID NO: 2), G227S and M228W (in the KS domain on SEQ ID NO: 3);
R90K (in the MPT domain on SEQ ID NO:1), G227S and M228W (in the KS domain on SEQ ID NO: 3);
increase(s) the selectivity for the production of C₆ fatty acids, C₆ fatty acid CoA esters, C₆ fatty acid ethyl esters, C₆ fatty acid esters with other metabolites, esters and/or enzyme bound C₆ fatty acids compared to wild type polypeptide(s) or the polypeptide without such amino acid substitution(s); and/or
increase(s) the selectivity for the production of C₈ fatty acids, C₈ fatty acid CoA esters, C₈ fatty acid ethyl esters, C₈ fatty acid esters with other metabolites, and/or enzyme bound C₈ fatty acids compared to wild type polypeptide(s) or the polypeptide without such amino acid substitution(s); and/or
increase(s) the selectivity for the production of C₁₀ to C₁₂ fatty acids, C₁₀ to C₁₂ fatty acid CoA esters, C₁₀ to C₁₂ fatty acid ethyl esters, C₁₀ to C₁₂ fatty acid esters with other metabolites, and/or enzyme bound C₁₀ to C₁₂ fatty acids compared to wild type polypeptide(s) or the polypeptide without such amino acid substitution(s).

### Nucleic acid molecules

As discussed above, the present invention provides a nucleic acid molecule, coding for a protein or polypeptide according to the present invention.

As discussed above, the present invention provides nucleic acid molecules, coding for the proteins or polypeptides according to the present invention.

As discussed above, the present invention provides a nucleic acid molecule, coding for a domain or subunit of a polypeptide/protein according to the present invention.

Preferably the nucleic acid molecules of the invention comprise or consist of
the nucleic acid sequences of SEQ ID NO: 4 coding for the MPT domain (with polypeptide sequence SEQ ID NO: 1), SEQ ID NO: 5 coding for the AT domain (with polypeptide sequence SEQ ID NO: 2) and/or SEQ ID NO: 6 coding for the KS domain (with polypeptide sequence SEQ ID NO: 3),
which comprise the respective nucleotide exchanges which lead to the amino acid substitution(s) of the present invention.

SEQ ID NO. 4 shows the nucleic acid sequence (part of gene *HMPREF0281_01212,* to be found in Genbank: ADNS01000009.1) coding for the MPT domain (SEQ ID NO: 1):

TCCATTTTGGAAATTGTACGCAACAACCCACCGCTGGTCGTTGTCGACGGCGAAGAGTTCACCCACCCAGATGGC
ACTCTATTCTTAACCCAGTTCACGCAGGTCGCGATGGCGACATTGGGCTGCGCCCAAATCGCGGAGCTGGCAGAA
GCCGGCGTGAAATCCCAGGACACTTACTTCGCCGGACACTCCGTGGGCGAATACAACGCCCTAGCTGCCTACGCA
CAGGTGCTGTCTTTGGAAGCCGTTGTAGAAATCGTCTACGCCAAGGGTTTGACCATGCACCGCCTGGTCGACCGT
GACGCAGAAGGCAACTCCAACTACGGCCTGGCAGCGCTGCGCCCGAATAAGATTGGCATCCGCGCCGAGGACGTC

TTTGACTACGTCGCGCAGGTCTCGCAGACCCGCGGCGAATTCCTAGAAATCGTCAACTACAACATCGCCGGTGTG
CAGTACGCCGTCGCCGGCACGCGAGCAGGGTTGGCAGCATTGGCTGCCGATGCCGAGTCGCGCGCGCCGGGCATG
CGCTCATTTATCATGATCCCGGGCATCGACGTGCCATTTCACTCTTCGCACCTGTTGGGCGGCGTCGATAACTTC
CGCACGCACCTGGACATGCTCATTCCAGACGACGTGGATTTGGATATTCTGCGCGGTCGCTACATCCCTAACCTG
GTGGCTCGCCCGTTTGAGCTCACCCGCGAATTCGTCGAAGCCATGGCCGATGTCGTCGATTCGACGTACGTCAAC
GACATCCTCGCCGACTTCGATAAGGCCACGGACGACCCGGCCAAGATGGGCCGCACCCTGCTCATTGAGCTCTTA
GCGTGGCAATTTGCCTCACCTGTGCGCTGGATCGAAACCCAAGACTTGCTGCTGTCTGCCCCAAGCGTCAGCGCC
GGAGCCGGCACCGCAGGTCTAGGCGTGGAGCGCTTTGTAGAAATCGGCGTGGGTTCTTCCCCGACGCTTGCGAAT
ATGCTCGGCCAAACCCTGCGCTTGCCGCAATACGCCGGCAATCCCATTGAGGTTCTCAACGTGGAA

SEQ ID NO: 5 shows the nucleic acid sequence (part of gene *HMPREF0281_01212,* to be found in Genbank: ADNS01000009.1) coding for the AT domain (SEQ ID NO: 2)

GTGACTATTGGCATCTCTAACCACCGCCTGGTGAACCGACTCGAGTCTGAAAAATTTGCTCTCAGCTTTTCCGGA
CAAGGTTTTAGCTGGTTGCCCACCTTGCGCACGGTAATTGCCGGTGGCAATGGCGCGCAGGTAGCCGGAGCAGTC
GATGCAGCGGGCAAGTTGCTGGAGCCAGTAGCAGATGAGCTCGTTGCTGCACTGCCACACGGCTTCGATCCGGTG
GAGTGGGCTGGCAACGCCGAGGACCCAGCGTGGGATTTGTCAGATTCGTCGATTTCTACCCCGGGCATCGTGGTC
GCCCAGTACGGCCTGATTGAGTCATTGAAAGCCCAGGGATTAGATCCTGCGGATGCAGTCGCACACATTGGCCAT
TCCCAAGGCGCGCTTGCTACTTATATCTCCTCCGGGCGCGCAGGCAGCAGAGGTTATTGCGCTGGCGCAGCTC
GCCGGCGTCGCTATTTCTAAGACCGCACGCGCCACGGACTCATCCGTATCGCTGCGGGTTCACCGATGGTCTCC
ATCGCCGGCATTTCCCGCGAGCAGCTGGCCAAGGTGCTGGCTAAAACCGGTGTCGATAGCGTTATTGGCCTGCAA
AATAACCACAACACTTTCGTTGTTACCGGCAGCCCGGAAGATAATAATAAGGTCATTGAGGCGATTGAAAAATTA
GCCGCCAAAGACGCGAAAGCGATTGAAAATAAAGAACGCGGCGGCAACCCCTTTGCCCCGCGTATTAATGCTTTG
CCGGTGCAATCTGGCTACCACCACCCAAAGATGTCCGCGGCAGTAGAGATGGTCACCAGCTGGGCAGAGCGCATC
GGGCTGGATAAAGACTTGGCTCACGATGGCGCAGAGGCAGTCTTGACGGAGATCGTGAACTGGCCTGCCACGGTG
CAGTCCGCTGTCGATGCGGGCGTGACGTGGATTTTGGAATGCGGTCCGGAAAAGGGCATGGCTGCCTTGACCAAG
GCGGTCGTCGCCGGTCAAGGCATTGGGGTGCAGGTTGTCTCCACGGACGAAGGCCAAGCAGCGCTTTTCGAT

SEQ ID NO: 6 shows the nucleic acid sequence (part of gene *HMPREF0281_01212,* to be found in Genbank: ADNS01000009.1)coding for the KS domain (SEQ ID NO: 3)

CTCGATGACATGGTGGTTATCGTCGGTGCCGGCGAAATTGGGCCGGTGGATCGTCTCGGACCCGTTTCGAGGTG
GAGATGACCGGTGACCTTACCGCCGCTGGTGTTGTCGAATTGGCGTGGACTACTGGCCTGATTGCCTGGGATGAT
GCCTCGGGTGCGTGGTTTGATGCCGAGGGCGAGGAAATCGCCGAGGAAGATATCTACGACCGCTTCCACGATGAA
GTCCTAGCCAACGTCGGCGTACGCAAGTACCACGATGACTACGGCCCGAAGATGCCGATGGTGGATAACTTGGCT
CCGGAGCTGACCACCATCTACCTCGAACGCGATATGACCTTTGCTGTTGCGGATGAAGAAACCGCGCGCACGTTT
GTTGCCGACGTGGAAGGTGCCACCGCGCACTTTGACGGCGAGGAATGGCAAGTCACCCGCCCAGCTGGGGCCGAG
GTGCGCGTGCCGCGGCGCGTGGCGATGACGCGCTTTGTCGGCGGGCAGATTCCGCAAGGGTTTGACCCCGGTGTC
TACGGTATTCCGGCCGATATGATTGACAACCTCGACCGGGTGGCCTTGTGGAACCTGGTGTGCACGGTGGATGCT
TTCTTGGCGGCTGGGTTTAGCCCCGCGGAATTGCTGGCGCATGTGCACCCGGCGCGCGTGAGCTCGACCCAGGGC
ACGAGTTGGGGCGGCATGCAGTCGATGCGCTCGCTGTACCTGGACAAGCTGCTGGCGCAGCCGCGCCCGAATGAC
ATCTTGCAGGAAGCACTGCCGAATGTCGTGGCAGCGCACGTCATGCAGTCTTATGTCGGCGGTTATGGCCAGATG
GTTCACCCGGTTGCCGCGTGTGCGACCGCGGCTGTCTCTGTCGAAGAGGGCATGGATAAACTGCGTTTGCGCAAG

GCTGATTTCGTGGTCGCCGGCGGTATTGATGACCTGTCGATTGAAGGCATTACGGGCTTCGGCGATATGGCGGCC
ACGGCGGATTCCGGCGAGATGGAAGCCAAGGGCATTGACCACAAGTACTTCTCGCGTGCCAATGACCGTCGTCGT
GGCGGGTTCATTGAGTCGGAAGGCGGCGGCACCATCTTGCTGGCGCGCGGTAGCTTGGCTGCGGAATTGGGCCTG
CCAGTCTTGGGCGTAGTTGGCTTCGCGGAGTCTTTCGCCGACGGTGCGCACACCTCCATTCCAGCGCCTGGTCTG
GGTGCGCTTTCTGCCGCGCGTGGTGGGCAGATTCCCGTCTAGCGCAGGGATTGGCCGAGCTTGGGGTGTCTGCG
AATGAAATCGCGGTCATTTCCAAGCACGACACGTCTACGAATGCCAATGACCCGAATGAGTCTGACCTGCATGAG
CGAATCGCAGCGTCAATGGGCCGCGATGCGGGCAACCCGTTGTTTATCATCTCGCAAAAGACCCTGACCGGTCAC
GCCAAGGGCGGTGCCGCAGCCTTCCAGCTGGTTGGTCTCACCCAGGTTCTGCGCAACGGTGTATTGCCACCGAAC
CGCTCGTTGGACTGTGTGGATCCGGCGTTGCGCCAGCACACTCATTTGACCTGGCTGCGCGAGCAGCTGGATTTG
CGTGCAACCCCACCAAAGGCCGGGCTGGTTACATCCTTGGGCTTTGGTCACGTCTCCGCGCTGGTTGCTGTGGTT
CACCCTGGCGCATTCGTGGAAGCT

In one embodiment, the nucleic acid molecule of the present invention further comprises:
- vector nucleic acid sequences, preferably expression vector sequences,
   and/or
- promoter nucleic acid sequences and terminator nucleic acid sequences,
   and/or
- comprises other regulatory nucleic acid sequence.

In one embodiment, the nucleic acid molecule of the present invention comprises dsDNA, ssDNA, cDNA, LNA, PNA, CNA, RNA or mRNA or combinations thereof.

The nucleic acid molecules according to the invention preferably comprise nucleic acid sequences, which are (except for the addition of the amino acid substitution(s) according to the invention) identical with the naturally occurring nucleic acid sequence or are codon-optimized for the use in a host cell.

The nucleic acid molecule used according to the present invention is preferably a nucleic acid expression construct.

Nucleic acid expression constructs according to the invention are expression cassettes comprising a nucleic acid molecule according to the invention, or expression vectors comprising a nucleic acid molecule according to the invention or an expression cassette, for example.

A nucleic acid expression construct preferably comprises regulatory sequences, such as promoter and terminator sequences, which are operatively linked with the nucleic acid sequence coding for the polypeptide(s) of the invention.

The nucleic acid expression construct may further comprise 5' and/or 3' recognition sequences and/or selection markers.

### Host cells

As discussed above, the present invention provides host cells containing a nucleic acid molecule according to the present invention.

Preferably, the host cells of the present invention express said nucleic acid molecule.

Preferably, a host cell according to the present invention is a bacterial cell.

The bacterial cell is more preferably a member of a genus selected from the group *Corynebacterium, Mycobacterium, Escherichia, Nocordia, Bacillus, Clostridium, Pseudomonas, Lactobacillus* or *Leuconostoc.*

The bacterial cell is more preferably a member of a species selected from the group of *(Corynebacterium glutamicum, Escherichia coli, Bacillus subtilis; Clostridium ljungdahlii*, *Pseudomonas putida; Lactobacillus bifermentans* or *Leuconostoc mesenteroides.*

In a preferred embodiment, the host cell belongs to the species *Escherichia coli.*

Preferably, a host cell according to the present invention is a fungus cell and more preferably a yeast cell.

The yeast cell is preferably a member of a genus selected from the group of *Saccharomyces* species, *Kluyveromyces* sp., *Hansenula* sp., *Arxula* sp., *Rhodosporidium* sp., *Pichia* sp. or *Yarrowia* sp.

The yeast cell is more preferably a member of a species selected from the group of S. *cerevisiae, S. bulderi, S. barnetti, S. exiguus, S. uvarum, S. diastaticus, K lactis, K marxianus, K. fragilis, H. polymorpha, P. pastoris* and *Y. lipolytica,*
such as *S. cerevisiae, K. lactis, H. polymorpha, P. pastoris* or *Y. lipolytica.*

Preferably, a host cell according to the present invention is an algae cell.

The algae cell is more preferably a member of a genus selected from the group *Chlamydomonas, Chlorella, Haematococcus, Dunaliella, , Nannochloropsis, Thalassiosira, Phaeodactylum, Porphyridium* or *Scenedesmus*

The algae cell is more preferably a member of a species selected from the group of *Chlamydomonas reinhardtii* or *Haematococcus pluvialis.*

Preferably, the host cell (preferably a bacterial cell) has an elevated overall production of short fatty acids, CoA esters of short fatty acids, ethyl esters of short fatty acids, esters of short fatty acids with other metabolites, and/or enzyme bound short fatty acids (C₆ to C₁₂) compared to a cell not containing a nucleic acid molecule according to the present invention.

In one embodiment, the host cell has an increased yield or increased selectivity in the production of *C₆ fatty acids,* C₆ fatty acid CoA esters, C₆ fatty acid ethyl esters, C₆ fatty acid esters with other metabolites, and/or enzyme bound C₆ fatty acids compared to a cell not containing a nucleic acid molecule according to the present invention.

The selectivity for the production of C₆ fatty acids, C₆ CoA esters and/or enzyme bound C₆ fatty acids compared to wild type is increased where the share of C₆ makes up at least 30%, or preferably at least 40% or 50% or 70% or 80% or 90% of the detected chain length between C₆ and C₁₂.

In one embodiment, the host cell has an increased yield or increased selectivity in the production of *C₈ fatty acids,* C₈ fatty acid CoA esters, C₈ fatty acid ethyl esters, C₈ fatty acid esters with other metabolites, and/or enzyme bound C₈ fatty acids compared to a cell not containing a nucleic acid molecule according to the present invention.

The selectivity for the production of C₈ fatty acids, C₈ CoA esters and/or enzyme bound C₈ fatty acids compared to wild type is increased where the share of C₈ makes up at least 30%, or preferably at least 40% or 50% or 70% or 80% or 89% of the detected chain length between C₆ and C₁₂.

In one embodiment, the host cell has an increased yield or increased selectivity in the production of *C₁₀ to C₁₂ fatty acids,* C₁₀ to C₁₂ fatty acid CoA esters, C₁₀ to C₁₂ fatty acid ethyl esters, C₁₀ to C₁₂ fatty acid esters with other metabolites, and/or enzyme bound C₁₀ to C₁₂ fatty acids compared to a cell not containing a nucleic acid molecule according to the present invention.

As discussed above, for example, proteins or polypeptides of the present invention, wherein the amino acid substitution(s) is/are selected from
I151A (in the AT domain on SEQ ID NO: 2), G227S and M228W (in the KS domain on SEQ ID NO: 3);
R90K (in the MPT domain on SEQ ID NO: 1), G227S and M228W (in the KS domain on SEQ ID NO: 3);
increase(s) the selectivity for the production of C₆ fatty acids, C₆ CoA, C₆ fatty acid ethyl esters, C₆ fatty acid esters with other metabolites, esters and/or enzyme bound C₆ fatty acids compared to wild type polypeptide(s) or the polypeptide without such amino acid substitution(s); and/or
increase(s) the selectivity for the production of C₈ fatty acids, C₈ fatty acid CoA esters, C₈ fatty acid ethyl esters, C₈ fatty acid esters with other metabolites, and/or enzyme bound C₈ fatty acids compared to wild type polypeptide(s) or the polypeptide without such amino acid substitution(s); and/or
increase(s) the selectivity for the production of C₁₀ to C₁₂ fatty acids, C₁₀ to C₁₂ fatty acid CoA esters, C₁₀ to C₁₂ fatty acid ethyl esters, C₁₀ to C₁₂ fatty acid esters with other metabolites, and/or enzyme bound C₁₀ to C₁₂ fatty acids compared to wild type polypeptide(s) or the polypeptide without such amino acid substitution(s).

### Methods and uses for producing fatty acids and/or biofuels and/or flavouring substances and/or fine chemicals,

As discussed above, the present invention provides a method for the production of short fatty acids, CoA esters of short fatty acids, short fatty acid ethyl esters, short fatty acid esters with other metabolites, and/or enzyme bound short fatty acids (C₆ to C₁₂).

Said method comprises the expression of a nucleic acid molecule according to the present invention, preferably in a host cell according to the present invention.

As discussed above, the present invention provides a method for the production of
- biofuels,
- flavoring compounds or substances
   and/or
- fine chemicals.

Said method comprises the expression of a nucleic acid molecule according to the present invention, preferably in a host cell according to the present invention.

Biofuels which can be produced with the method are, for example, short alkanes, short alkenes, short alkynes, methyl ketones, short esters or alcohols.

Flavoring compounds or substances which can be produced with the method are, for example, fatty acids esterified with short alcohols or esters from short fatty acids (C₆ to C₁₂).

Fine chemicals which can be produced with the method are, for example, natural compounds, where preferably short fatty acids (C₆ to C₁₂) or their derivatives (such as CoA esters, methyl/ethyl esters, esters with other metabolites, alcohols) are used as building block(s).

As discussed above, the present invention provides the use of
- a protein or polypeptide according to the present invention,
- a nucleic acid molecule according to the present invention, or
- a host cell according to the present invention,
   for
- the production of short fatty acids (C₆ to C₁₂), CoA esters of short fatty acids, short fatty acid ethyl esters, short fatty acid esters with other metabolites, enzyme bound short fatty acids.

In particular, the present invention provides the use of a protein or polypeptide according to the present invention, a nucleic acid molecule according to the present invention, or a host cell according to the present invention, for
- the bulk and/or specific production of short fatty acids (C₆ to C₁₂),
- the bulk and/or production of CoA esters of short fatty acids (C₆ to C₁₂),
- the bulk and/or production of ethyl esters of short fatty acids (C₆ to C₁₂),
- the bulk and/or production of short fatty acids (C₆ to C₁₂) esters with other metabolites,
- the bulk and/or production of enzyme bound short fatty acids (C₆ to C₁₂).

As discussed above, the present invention further provides the use of
- a protein or polypeptide according to the present invention,
- a nucleic acid molecule according to the present invention, or
- a host cell according to the present invention,
for
- the production of biofuels, such as short alkanes, short alkenes, short alkynes, methyl ketones, short esters or alcohols,
- the production of fine chemicals, such as natural compounds where preferably short fatty acids (C₆ to C₁₂) or their derivatives (such as CoA esters, methyl/ethyl esters, esters with other metabolites, alcohols) are used as building block(s), or
- the production of flavoring compounds or substances, such as esters from short fatty acids (C₆ to C₁₂).

### Further description of preferred embodiments

The study shown here, is the first one reported where by rational design *C*. *ammoniagenes FAS* was modified to produce short fatty acids. It was possible to show significant increase in the production of short fatty acids in the range of C₆ to C₁₂ in an *in vitro* set up to 32-fold higher than the wild type (13.8 mM instead of 0.4 mM). Besides overall production, the share of C₈-CoA was increased in one mutant to make up 47% of the total acyl-CoA products detected (C₆-C₁₈-CoA).

### - Results

The 1.9 MDa *C. ammoniagenes* FAS is accessible in high amounts from recombinant expression in *E. coli.* Phosphopantetheinylation was achieved by co-expression with its phosphopantetheine transferase AcpS. Chromatographic profiles and protein specific activity measurements indicated high protein quality (Table 3) (Stuible et al., 1997). X-ray crystallographic and cryo-electron microscopic data on fungal and bacterial FAS have recently documented a high structural homology of microbial FAS type I systems (Boehringer et al., 2013). As structural data at 3.1 Å and enzyme kinetic data are available, *S. cerevisiae* FAS was used for structural models (Jenni et al., 2007; Johansson et al., 2008).

FAS from C. *ammoniagenes* (Uniprot Accesion No.: D5NXL2) was engineered to produce short fatty acids in form of their CoA ester (C₆-C₁₂-CoA; where in Cₙ, n represents the number of carbons in the fatty acid chain) instead of the native long chain products palmitoyl-CoA (C₁₆-CoA) and stearoyl-CoA (C₁₈-CoA) (Kawaguchi et al., 1980).

Some examples of chain length manipulation have been reported in polyketide synthase systems, an enzyme class closely related to FAS: In the type III PKS octaketide synthase (OKS), a glycine was identified which, when mutated to bigger residues, only allows formation of shorter products (Abe et al., 2005). Further, structure-guided mutagenesis leading to increased chain length has been reported for the chain length factor CLF of the actinorhodin type II PKS complex (Tang et al., 2003).
To our knowledge, rational mutagenesis to control chain length in type I FAS has not previously been reported for bacterial FASs to which the *C. ammoniagenes* FAS belongs.

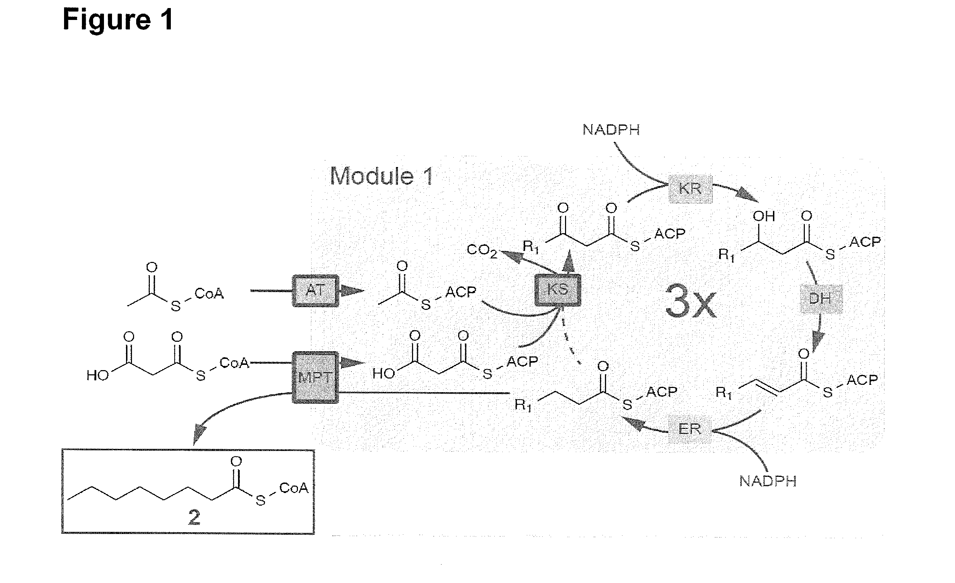

For chain length control in *C. ammoniagenes* FAS, modifications on several domains, namely KS, MPT and AT, were introduced (Fig. 1).

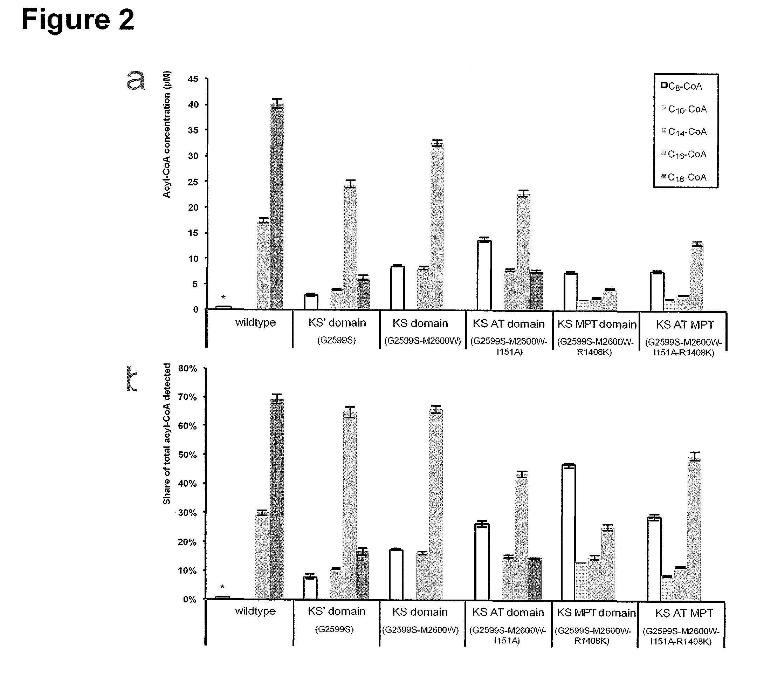

KS domain: The KS domain is responsible for elongation, and its manipulation was considered promising for chain length control. We intended to increase the yield of short fatty acids, such as C₈-CoA, by disfavoring the loading of the KS domain with longer acyl chains and preventing their elongation. Structural data of *S. cerevisiae* FAS show M1251 of the *S*. *cerevisiae* FAS in a central position within the binding channel in the KS domain. It is found in two conformations: directed into the KS channel, with the outer part of the channel accessible for substrate binding, and forced aside by long acyl chain making also the bottom of the KS channel accessible. The adjacent G1250 was suggested to contribute conformational freedom for the repositioning of M1251 of *S. cerevisiae* FAS (Johansson et al., 2008). In HsmtKAS, homologous to the KS domain, the methionine was linked to a bimodal product distribution (Christensen et al., 2007). Presumably owing to the energy penalty for rearrangement of the methionine, weak binding to HsmtKAS was specifically found for C₈-ACP (Zhang et al., 2005). Based on these previously reported findings, we sought to employ a gatekeeping function of M228 and constructed the *C. ammoniagenes* FAS mutants G227S (FAS^{G227S}, *C. ammoniagenes* FAS numbering) and G227S/M228W (FAS^{G227S-M228W}). In addition to the reported effect of the glycine to serine mutation, we reasoned that a bulkier tryptophan residue in place of methionine would be expected to counteract more efficiently the binding of long fatty acids in the KS binding channel. Both mutants showed an increase in the production of shorter fatty acids (Fig. 2).
Also, a third promising position in the KS was mutated based on sequence alignments with organisms known to produce C₆ at least as an intermediate, such as *Aspergillus parasiticus* and *Aspergillus flavus* (Hitchman et al., 2001), the L256Y mutation.

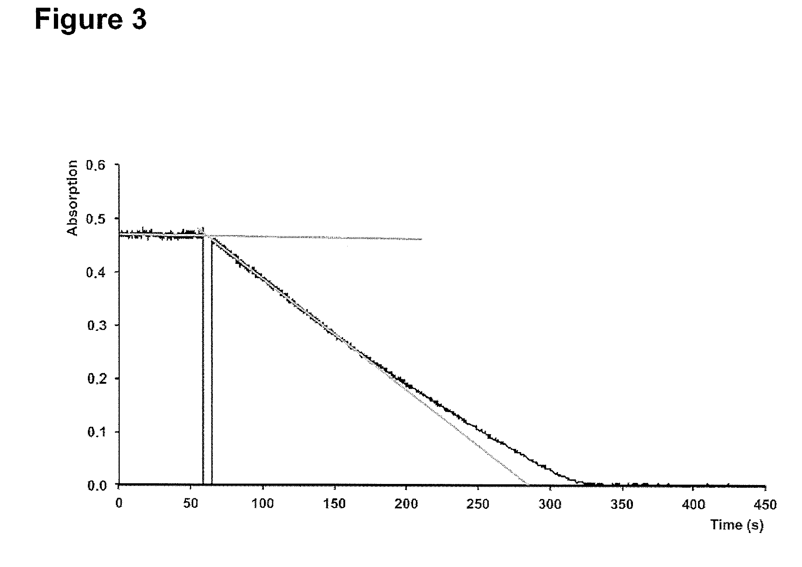

MPT domain: The MPT domain mediates the dual function of loading malonyl into the enzyme and releasing the acyl product as CoA-ester. Accordingly, malonyl-CoA (loading) and acyl-ACP (release) bind competitively to the same active site. In fungal FAS, the MPT domain has been characterized as accepting a broad spectrum of substrates (Pirson et al., 1973). We initially indented to shift the product spectrum towards shorter chain length by increasing the affinity of the MPT domain towards short fatty acids, facilitating their release. Several candidate mutations, evaluated by molecular modeling, did not show the desired effects *in vitro.* Finally, we succeeded in an inverse approach designed for favoring the acyl transfer by decreasing the affinity of malonyl-CoA by mutating the carboxyl coordinating R90 (Oefner et al., 2006). When combined with the G227S-M228W mutations in the KS domain, the triple mutant FAS^{G227S-M228W-R90K} yielded C₈-CoA that makes up 47% of the total acyl-CoA products detected (see Fig. 3). For mutations R90 to alanine (FAS^{G227S-M228W-R90A}), glycine (FAS^{G227S-M228W-R90G}) and glutamine (FAS^{G227S-M228W-R90Q}) (Bunkoczi et al., 2009) fatty acid synthesis was abolished.

AT domain: We introduced mutation I151A, (FAS^{G227S-M228W-I151A}) for increasing acetyl loading (Bunkoczi et al., 2009), and supporting the priming of fatty acid synthesis (see Fig. 1) (Kawaguchi et al., 1980). In product analysis, the AT domain mutation I151A, in combination with the KS domain mutations described above, led to higher absolute and relative yields of short fatty acids (see Fig. 2). However, neither KS/AT-mutated FAS^{G227S-M228W-I151A}, nor KS/MPT/AT-mutated FAS^{G227S-M228W-I151A-R90K} showed improved relative yields as compared to KS/MPT-mutated FAS^{G227S-M228W-R90K} (see Fig. 2).

### - Discussion

The FAS from *C*. *ammoniagenes* was manipulated for acyl chain length control with the introduction of mutations in the KS, in the AT domain and in the MPT domain, shifting the product spectrum towards shorter fatty acids. In sum, only with few mutations we were able to change the fatty acid synthesis product spectra. This was possible by employing key mutations that address catalytic activities, as well as by taking advantage of inherent broad substrate tolerance (of the MPT domain in transferring short fatty acids).

This concept of the specific mutations in the mentioned domains can easily be applied to other FAS systems. An overview of potentially promising FASs is shown in Table 1 (the numbering in this table refers to the amino acids as they are listed in the corresponding Uniprot Identifiers; for *C. ammoniagenes* FAS numbering according to Uniprot is shown marked with *, as well as numbering used in this text is shown indicated by #). Besides the specific examples listed in Table 1, a longer list of potential FAS candidates for this type of engineering are listed in Table 2, with their sequence identities of their Uniprot sequence in comparison to the entire polypeptide sequence of *C*. *ammoniagenes* FAS (Uniprot Accession No. D5NLX2).

It should be mentioned that the modified FAS can be employed in a system of microbial production of fatty acids of short length, as e.g. for biofuel production.(Torella et al., 2013). Recently, the generation of relevant compounds such as short chain alkanes, alcohols and fatty acids in *E. coli* has been reported.(Choi et al., 2013) Also type I systems have been engineered for synthesis of short fatty acids (Leber et al., 2014) but this system has disadvantages, such as the production of free fatty acids and a generally low yield compared to the strength of the employed promoter. In this context, the mutations analyzed in FAS *C*. *ammoniagenes* might be of wider relevance.

**Table 3: Statistics on enzymatic activities of FAS constructs.**

| Given values were received from different protein preparations (expression batches) as indicated. Sample mean (SEM) with standard deviation of the average values of the three batches. When three or more values have been recorded for one batch, the standard deviation is also indicated. Activities are shown in mU/mg of protein (1 U was defined as the incorporation of 1 µmol of malonyl-CoA per minute). For FAS constructs, activity was determined by monitoring NADPH consumption. | | | |
|---|---|---|---|
| | Wild type | FAS ^{G227S-M228W} | FAS^{G227S-M228W-I151A-R90K} |
| Batch 1 | 362 ± 55 | 158 | 43 |
| 2 | 210 ± 68 | 140 | 86 |
| 3 | 284 ± 28 | 109 | |
| SEM | 285 ± 76 | 135 | 64 |

The following examples and drawings illustrate the present invention without, however, limiting the same thereto.

### BRIEF DESCRIPTION OF THE DRAWINGS

**Figure 1****.** *Reaction scheme for the synthesis of short fatty acids.*
   The FAS variant carries modifications in the KS, AT and MPT domain to produce short fatty acids, such as C₈-CoA **2** from acetyl-CoA, malonyl-CoA and NADPH (instead of its native product, C₁₆- or C₁₈-CoA). Product analysis was conducted *in vitro* with the purified *C. ammoniagenes* FAS protein and the mentioned substrates, followed by extraction and eventual quantification via HPLC-UV (absorption at 260 nm).
**Figure 2****.** *Product spectra* of *C. ammoniagenes FAS variants.*
   FAS variants are short termed according to the domains that have been mutated. Specific mutations are given in parenthesis. (**a**) Concentration of acyl-CoAs of indicated chain lengths in the product assay. Bars marked with an asterisk are based on values received by extrapolation (values outside of calibration range). Octanoyl-CoA (C₈-CoA) output was increased 32-fold (G227S-M228W-I151A-variant in comparison to the wild type). (b) Percentage of molar concentrations of specific acyl-CoA in total acyl-CoAs. Here, also the share of C₈-CoA out of all detected CoA species was increased from 0.7% (wild type) to 47% (for the G227S-M228W-R90K-variant)
**Figure 3****.** *Exemplary result from activity assay.*
   NADPH decrease was monitored at 334 nm over time. After a period of constant absorption, the reaction is started by the addition of malonyl-CoA. Linear trends for both slopes, before and after the reaction, are used for are used for the determination of enzymatic activity.
**Figure 4****.** *Glycerol influence on product spectrum.*
   The product spectrum of FAS^{G227-M228W} as freshly prepared protein and as received from a glycerol stock. For the sample with glycerol, its final glycerol content in the assay was 1.2%.

### EXAMPLES

### 1. Materials and Methods

### 1.1 Cloning

For the initial FAS construct, *Corynebacterium ammoniagenes* genomic DNA (ordered from DSMZ, DSM 20306) was used. The construct was cloned into a pET-22b(+)vector (Novagen, Merck Millipore, Darmstadt, Germany) with an N-terminal strep twin tag with short linker (MSAWSHPQFEKGGGSGGGSGGSAWSHPQFEKGAGS) [SEQ ID NO. 8] or the shorter version, the strep II tag (MSAWSHPQFEKGAGS) [SEQ ID NO. 9] (for primers, see below). For preparation, the pET-22b(+)vector was digested with BamHI and XhoI and the insert was cloned using the Infusion HD cloning kit (Clontech, Mountain View, USA).
Point mutations were introduced using site-directed mutagenesis with forward and reverse primers that carried the mutation (see below). After extraction from a preparative agarose gel (0.8%), the linear fragment was ligated using the Infusion HD cloning kit (Clontech, Mountain View, USA). The vectors were transformed into competent cells (Stellar cells, Clontech) and amplified. All constructs were Sanger-sequenced. For co-transformation, the *C*. *ammoniagenes* FAS AcpS was amplified (for primers, see below) and cloned into a pETcoco vector (Novagen, Merck Millipore, Darmstadt, Germany).

### - Primers Module 1

Primers for the amplification of the *C*. *ammoniagenes* FAS for Infusion cloning into pET-22b(+)vector (15 bp overlap with vector ends at the 5' end, underlined). The amplified insert included approximately 270 bp after the actual stop codon as an artifact from initial cloning.
fwd 5' AAAAGGCGCCGGATCCACTATTGGCATCTCTAACCACCGCCTGG 3' [SEQ ID NO. 10]
rev 5' GGTGATGATGCTCGAGCTGGTGGCTTGCCGTAGATCGCTTGC 3' [SEQ ID NO. 11]

Primers for the introduction of point mutations were either designed as complimentary pairs with the mutation at a central position (PCR was then performed covering the whole vector). As alternative the overlap of the primers was approx. only 15 bp. The underlined part is indicating the overlap of the primers, the bold part shows the mutation site.
Primers covering the mutation in the AT domain (I151A)
   fwd 5' CAGCTC**GCC**GGCGTCGCTATTTCTAAG 3' [SEQ ID NO. 12]
   rev 5' GACGCC**GGC**GAGCTGCGCCAGCGCAATAAC 3' [SEQ ID NO. 13]
Primers covering the mutation in the MPT domain (R90K)
   fwd 5' GAAATCGTCTACGCC**AAG**GGTTTGACCATGCAC 3' [SEQ ID NO. 14]
   rev 5' GGCGTAGACGATTTCTACAACGGCTTCC 3' [SEQ ID NO. 15]
Primers covering the mutation in the KS domain (G227S)
   fwd 5' GCTCGACCCAGGGCACG**AGT**ATGGGCGGCATGCAGTCG 3' [SEQ ID NO. 16]
   rev 5' CGACTGCATGCCGCCCAT**ACT**CGTGCCCTGGGTCGAGC 3' [SEQ ID NO. 17]
Primers covering the double mutation in the KS domain (G227S-M228W)
   fwd 5' CCCAGGGCACG**AGTTGG**GGCGGCATGCAGTCGATGCGC 3' [SEQ ID NO. 18]
   rev 5' GCGCATCGACTGCATGCCGCC**CCAACT**CGTGCCCTGGG 3' [SEQ ID NO. 19]
Primers covering the double mutation in the KS domain (L256Y)
   fwd 5' ATC TTG CAG GAA GCA **TAT** CCG AAT GTC GTG 3' [SEQ ID NO. 20]
   rev 5' TGC TTC CTG CAA GAT GTC ATT CGG 3' [SEQ ID NO. 21]

### - Primers for the C. ammoniagenes FAS AcpS

The overlap to the vector is underlined, the parts binding the genomic DNA for the insert amplification are shown in bold.
fwd 5' AGAAGGAGATATAAGC**ATGCTCGACAACCGTGAAGCGATGAC** 3' [SEQ ID NO. 22]
rev 5' TCGAGTGCGGCCTAGG**TTACCGCTGGTACCGCAGCAGG** 3' [SEQ ID NO. 23]

### 1.2 Expression

For expression, the plasmid containing the FAS construct and the plasmid containing the AcpS were co-transformed into *E. coli* BL21 (DE3) gold competent cells (Agilent Technologies, Santa Clara, USA) according to the manufacture's protocol. Cells were plated on LB+1.5% agar (containing 50 µg/ml ampicillin, 11 µg/ml chloramphenicol, 0.01% arabinose) and grown over night at 37 °C. Five clones were picked randomly, then united in one pre-culture (35 ml LB media, 100 µg/ml ampicillin, 34 µg/ml chloramphenicol, 0.01% arabinose) and incubated at 200 rpm at 37 °C overnight. For the main culture, the pre-culture was transferred into 2 L TB media (containing 100 µg/ml ampicillin, 34 µg/ml chloramphenicol, 0.01% arabinose). The main culture was incubated at 180 rpm at 37 °C until OD₆₀₀ 0.8 to 1.0. After cooling to 20 °C, expressions were induced with IPTG (final concentration 250 µM). Expressions were performed at 20 °C overnight. Then, after centrifugation at 7000 rcf for 14 min, the supernatant was discarded and the cell pellet was used directly for protein purification or stored at -80 °C after freezing in liquid N₂.

### 1.3 Protein Purification

For protein purification, the cell pellet from 1 L TB culture (approx. 20 g) was resuspended in buffer W (100 mM Na₂HPO₄/NaH₂PO₄, pH = 7.2, 100 mM NaCl, 1 mM EDTA) to a total volume of 35 ml. DNAse I (2 mg; AppliChem, Darmstadt, Germany) and protease inhibitor (complete EDTA-free, Roche, Mannheim, Germany) were added).

Cells were broken using French press (16 000 psi, 1 100 bar). To avoid protein degradation, all following steps were carried out at 4 °C. After centrifugation for 1 hour at 60,000 rcf, the supernatant (approx. 30 ml) was put on a strep column with a 5 ml matrix volume (purchased at IBA, Goettingen, Germany). After washing with 8 column volumes (CV) of buffer W, the protein was eluted with 3 CV of buffer E (same as buffer W but with 2.5 mM D-Desthiobiotin). The fractions were checked for impurities by SDS-PAGE and then concentrated in a centrifugal filter with a 100,000 nominal molecular weight limit (Amicon Ultra-4, Merck Millipore, Ireland). The sample was further purified by size exclusion chromatography (column: Superose 6 10/300GL, GE Healthcare, buffer G: 100 mM Na₂HPO₄/NaH₂PO₄, pH = 7.2, 100 mM NaCl) and examined for its oligomeric state. Fractions were pooled and concentrated to a final concentration of 10 - 20 mg/ml of protein. Overall protein yields varied from 3 -18 mg per liter TB culture with a typical yield of 10 mg on average. A loss of activity was observed after the final purification step, most likely due to loss of FMN; a phenomena that has been described earlier (Morishima et al., 1982). Therefore, the samples were incubated with FMN in a five-fold molecular excess for 3 - 5 hours. By this procedure, the activity could be restored. For storage, glycerol was added to a final volume of 50% and the protein sample was stored at -20 °C until used.

### 1.4 Activity Assay

Activity assays on module 1, based on the previously reported assay from Lynen ENREF 43 (Lynen et al., 1969), were prepared on 60 µL scale including 30 µL of buffer AB (400 mM KH₂PO₄/K₂HPO₄, pH = 7.3, 3.5 mM DTT), 50 nmol acetyl-CoA, 30 nmol NADPH and 25 µg of the FAS protein. The reaction was incubated for two minutes at room temperature. After one minute of recording the absorption at 334 nm, the reaction was started by the addition of 60 nmol malonyl-CoA. For a typical result, see Fig. 3.

### 1.5 Product Assay Setup

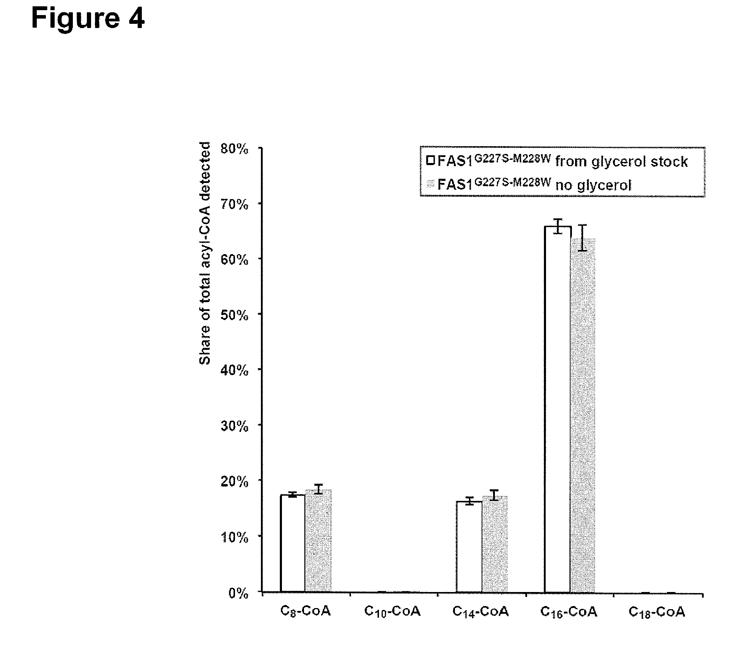

The product assay of module 1 was prepared on a 100 µL scale including 50 µL of buffer AB (400 mM KH₂PO₄/K₂HPO₄, pH = 7.3, 3.5 mM DTT), 20 nmol acetyl-CoA, 100 nmol malonyl-CoA, 225 nmol NADPH and 20 µg of the FAS protein. CoA ester analysis was conducted with proteins from 50% glycerol stock that was stored at -20 °C. Glycerol did not have an influence on the product distribution (Fig. 4).

The assays were left to react overnight. An internal standard (IS) was added; for the analysis of CoA esters (module 1) it was isovaleryl-CoA (iC₅-CoA) and *n*-heptadecanoyl-CoA (C₁₇-CoA) (both acquired from Sigma-Aldrich). In order to stop the reaction and to precipitate the protein, four volumes of acetone (cooled to -20 °C) were added directly after IS addition. The mixture was vortexed for 20 sec and kept at -20 °C for 1 hour to complete the precipitation process. After centrifugation (5 min at 20,000 rcf), 4/5 of the supernatant were transferred to a new vial and the solvent evaporated to dryness at 4 °C under reduced pressure in a SpedVac. A volume of 60 µL water was added. The samples were treated 5 min in an ultrasonification bath and then were ready to be measured by HPLC-UV-MS.

### 1.6 HPLC quantification

### 1.6.1 LC-MS analysis of acyl-CoAs

LC-MS analysis of acyl-CoAs was carried out using a Dionex Ultimate 3000 RSLC coupled to a Bruker micrOTOF-Q II equipped with an electrospray ionization source. Chromatographic separation was performed on a RP-18 column (100 x 2.1 mm, particle size 1.7 µm, Waters Acquity BEH) with a mobile-phase system consisting of buffer A (water, 10 mM triethylamine/acidic acid buffer, adjusted to pH = 9.0) and buffer B (acetonitrile) based on a method previously described (Mauriala et al., 2004) A multistep gradient at a flow rate of 0.25 mL min⁻¹ was used with the starting condition of buffer B at 7%, a linear increase to 60% until 6 min, then to 70% until 9.5 min and finally to 90% until 10 min runtime. Data were acquired in negative mode in a scan range from 200-2000 *m*/*z* and later analyzed using DataAnalysis 4.0 software (Bruker Daltonik GmbH). For the quantification, the UV trace at 260 nm was used. Calibration was done for C₆-CoA, C₈-CoA, C₁₀-CoA, C₁₄-CoA, C₁₆-CoA, C₁₈-CoA as well as for the internal standards iC₅-CoA and C₁₇-CoA (see Table 4 below for details). The concentration in the sample was determined and initial concentration in the assay was calculated to the IS reference.

### Quantification of CoA esters by HPLC

For acyl-CoA analysis, the recovery of the extraction procedure was tested with the internal standards as representatives. Three different concentrations (20 µM, 50 µM, 100 µM) with four replicates for every concentration for extracted and non-extracted controls each were measured. The obtained rates for iC₅-CoA were (74.5±0.9)% for 20 µM, (74.8±1.4)% for 50 µM; (73.1±1.7)% for 100 µM respectively. For C₁₇-CoA the rates were (70.1±1.8)% for 20 µM, (75.0±2.0)% for 50 µM; (72.4±1.9)% for 100 µM respectively.
The calibration coefficients and calibration ranges are listed for each CoA species. For the shorter acyl-CoAs (C₆-CoA, C₈-CoA, C₁₀-CoA), iC₅-CoA served as the internal reference to calculate loss from the work up procedure, for the longer acyl-CoA (C₁₄-CoA, C₁₆-CoA, C₁₈-CoA) C₁₇-CoA was used.
Concentrations for the calibration were 5 µM, 10 µM, 15 µM, 20 µM, 25 µM, 50 µM, 100 µM, 150 µM and 200 µM with three replicates each (used concentrations for the calibration are indicated by the calibration range). The signal of all points included in the calibration range differed from their calculated value no more than 15% (20% for the lower limit of quantification).

**Table 4: Characteristics of the calibration for acyl-CoA esters.**

| UV detection at 260 nm was used for quantification. For each calibration point three replicates were recorded. | | | | |
|---|---|---|---|---|
| Compound | slope (m) | intercept (c) | Correlation coefficient (R²) | Calibration range |
| iC₅-CoA | 25.94 | -23.83 | 1.0000 | 5 - 200 µM |
| C₁₇-CoA | 19.74 | -104.31 | 0.9981 | 5 - 200 µM |
| C₆-CoA | 20.52 | 76.38 | 0.9981 | 10 - 200 µM |
| C₈-CoA | 27.11 | 1.77 | 1.0000 | 5 - 200 µM |
| C₁₀-CoA | 18.29 | -0.45 | 0.9998 | 5 - 100 µM |
| C₁₄-CoA | 27.06 | -37.69 | 0.9993 | 5 - 150 µM |
| C₁₆-CoA | 27.64 | -44.87 | 0.9997 | 5 - 200 µM |
| C₁₈-CoA | 24.62 | -182.83 | 0.9992 | 15 - 150 µM |

The features disclosed in the foregoing description, in the claims and/or in the accompanying drawings may, both separately and in any combination thereof, be material for realizing the invention in diverse forms thereof.

### REFERENCES

Abe I, Oguro S, Utsumi Y, Sano Y, & Noguchi H (2005) Engineered biosynthesis of plant polyketides: chain length control in an octaketide-producing plant type III polyketide synthase. Journal of the American Chemical Society 127(36):12709-12716.
Beld J, Lee DJ, & Burkart MD (2015) Fatty acid biosynthesis revisited: structure elucidation and metabolic engineering. Molecular BioSystems 11(1):38-59.
Boehringer, D., Ban, N. & Leibundgut, M. 7.5-Å Cryo-EM Structure of the Mycobacterial Fatty Acid Synthase. Journal of Molecular Biology 425, 841-849
Bunkoczi G, et al. (2009) Structural Basis for Different Specificities of Acyltransferases Associated with the Human Cytosolic and Mitochondrial Fatty Acid Synthases. Chemistry & Biology 16(6):667-675.
Choi YJ & Lee SY (2013) Microbial production of short-chain alkanes. Nature 502(7472):571-574.
Christensen CE, Kragelund BB, von Wettstein-Knowles P, & Henriksen A (2007) Structure of the human β-ketoacyl [ACP] synthase from the mitochondrial type II fatty acid synthase. Protein Science 16(2):261-272.
Hitchman TS, et al. (2001) Hexanoate Synthase, a Specialized Type I Fatty Acid Synthase in Aflatoxin B1 Biosynthesis. Bioorganic Chemistry 29(5):293-307.
Jenni S, et al. (2007) Structure of Fungal Fatty Acid Synthase and Implications for Iterative Substrate Shuttling. Science 316(5822):254-261.
Kawaguchi A, Arai K, Seyama Y, Yamakawa T, & Okuda S (1980) Substrate Control of Termination of Fatty Acid Biosynthesis by Fatty Acid Synthetase from Brevibacterium ammoniagenes TI. The Journal of Biochemistry 88(2):303-306.
Leber C & Da Silva NA (2014) Engineering of Saccharomyces cerevisiae for the synthesis of short chain fatty acids. Biotechnology and Bioengineering 111(2):347-358.
Leber C, Polson B, Fernandez-Moya R, & Da Silva NA (2015) Overproduction and secretion of free fatty acids through disrupted neutral lipid recycle in Saccharomyces cerevisiae. Metabolic Engineering 28(0):54-62.
Liu H, et al. (2012) Production of extracellular fatty acid using engineered Escherichia coli. Microbial Cell Factories 11.
Lynen F (1969) [3] Yeast fatty acid synthase. Methods in Enzymology, ed John ML (Academic Press), Vol Volume 14, pp 17-33.
Mauriala T, Herzig KH, Heinonen M, Idziak J, & Auriola S (2004) Determination of long-chain fatty acid acyl-coenzyme A compounds using liquid chromatography-electrospray ionization tandem mass spectrometry. Journal of Chromatography B 808(2):263-268.
Morishima N, Ikai A, Noda H, & Kawaguchi A (1982) Structure of bacterial fatty acid synthetase from brevibacterium ammoniagenes. Biochimica et Biophysica Acta (BBA) - Protein Structure and Molecular Enzymology 708(3):305-312.
Oefner C, Schulz H, D'Arcy A, & Dale GE (2006) Mapping the active site of Escherichia coli malonyl-CoA-acyl carrier protein transacylase (FabD) by protein crystallography. Acta Crystallographica Section D 62(6):613-618.
Pirson W, Schuhmann L, & Lynen F (1973) The Specificity of Yeast Fatty-Acid Synthetase with Respect to the "Priming" Substrate. Decanoyl-CoA and Derivatives as "Primers" of Fatty-Acid Synthesis in vitro. European Journal of Biochemistry 36(1):16-24.
Peralta-Yahya, P. P., Zhang, F., del Cardayre, S. B. & Keasling, J. D. (2012) Microbial engineering for the production of advanced biofuels. Nature 488: 320-328.
Runguphan W & Keasling JD (2014) Metabolic engineering of Saccharomyces cerevisiae for production of fatty acid-derived biofuels and chemicals. Metabolic Engineering 21(0):103-113.
Stuible H-P, Meurer G, & Schweizer E (1997) Heterologous Expression and Biochemical Characterization of two Functionally Different Type I Fatty Acid Synthases from Brevibacterium Ammoniagenes. European Journal of Biochemistry 247(1):268-273.
Tang Y, Tsai SC, & Khosla C (2003) Polyketide chain length control by chain length factor. Journal of the American Chemical Society 125(42):12708-12709.
Tehlivets O, Scheuringer K, & Kohlwein SD (2007) Fatty acid synthesis and elongation in yeast. Regulation of Lipid Metabolism in Yeast 1771(3):255-270.
Torella JP, et al, (2013) Tailored fatty acid synthesis via dynamic control of fatty acid elongation. Proceedings of the National Academy of Sciences 110(28):11290-11295.
Zhang L, Joshi AK, Hofmann J, Schweizer E, & Smith S (2005) Cloning, Expression, and Characterization of the Human Mitochondrial β-Ketoacyl Synthase: COMPLEMENTATION OF THE YEAST CEM1 KNOCK-OUT STRAIN. Journal of Biological Chemistry 280(13):12422-12429.

## Claims

1. A *protein* involved in fatty acid synthesis,
said protein comprising one or more polypeptide chains, wherein said polypeptide chain(s) comprise
(i) one or more subunits comprising the amino acid sequences of
SEQ ID NO: 1 (malonyl/palmitoyl transferase domain, MPT domain);
SEQ ID NO: 2 (acetyl transferase domain, AT domain), and/or
SEQ ID NO: 3 (ketoacyl synthase domain, KS domain);
*(ii)* at least one amino acid substitution
- in the MPT domain at a position corresponding to R90 of the amino acid sequence of SEQ ID NO: 1;
- in the AT domain at a position corresponding to 1151 of the amino acid sequence of SEQ ID NO: 2;
and/or
- in the KS domain, preferably in the acyl binding channel, preferably selected from a position corresponding to G227, M228 and L256 of the amino acid sequence of SEQ ID NO: 3;
wherein the amino acid sequence comprising the at least one amino acid substitution has at least 50%, or preferably at least 60% or 70% or 80% or 90% or 95% sequence identity to the respective amino acid sequence of SEQ ID NO: 1, 2 and/or 3.

2. The protein according to claim 1, wherein the protein is type I FAS of *Corynebacterium ammoniagenes,* more preferably the FAS having the amino acid sequence of SEQ ID NO. 7.

3. A polypeptide domain comprising
(i) one or more subunits comprising the amino acid sequences of
SEQ ID NO: 1 (malonyl/palmitoyl transferase domain, MPT domain);
SEQ ID NO: 2 (acetyl transferase domain, AT domain), or
SEQ ID NO: 3 (ketoacyl synthase domain, KS domain);
*(ii)* at least one amino acid substitution
- in the MPT domain at a position corresponding to R90 of the amino acid sequence of SEQ ID NO: 1;
- in the AT domain at a position corresponding to 1151 of the amino acid sequence of SEQ ID NO: 2;
and/or
- in the KS domain, preferably in the acyl binding channel, preferably selected from a position corresponding to G227, M228 and L256 of the amino acid sequence of SEQ ID NO: 3;
wherein the amino acid sequence comprising the at least one amino acid substitution has at least 50%, or preferably at least 60% or 70% or 80% or 90% or 95% sequence identity to the respective amino acid sequence of SEQ ID NO: 1, 2 and/or 3.

4. The protein or polypeptide domain according to any one of claims 1 to 3, comprising the amino acid substitution(s) R90K in the MPT domain (SEQ ID NO. 1), and/or I151A in the AT domain (SEQ ID NO: 2).

5. The protein or polypeptide domain according to any one of claims 1 to 4, comprising the amino acid substitution(s) in the acyl binding channel of the KS domain, preferably comprising amino acid substitution(s) G227S, M228W and/or L256Y in the KS domain (SEQ ID NO: 3).

6. The protein according to any one of claims 1 to 5, selected from the group of
- a protein comprising the amino acid substitution G227S;
- a protein comprising the amino acid substitutions G227S and M228W;
- a protein comprising the amino acid substitutions I151A, G227S and M228W;
- a protein comprising the amino acid substitutions R90K, G227S and M228W; and
- a protein comprising the amino acid substitutions I151A, R90K, G227S and M228W;
wherein
R90K refers to R90K in the MPT domain of the amino acid sequence of SEQ ID NO: 1; I151A refers to I151A in the AT domain of the amino acid sequence of SEQ ID NO: 2; G227S refers to G227S in the KS domain of the amino acid sequence of SEQ ID NO: 3; M228W refers to M228W in the KS domain of the amino acid sequence of SEQ ID NO: 3; and
L256Y refers to L256Y in the KS domain of the amino acid sequence of SEQ ID NO: 3.

7. The protein according to any one of claims 1 to 6, selected from
variant G227S;
variant G227S / M228W;
variant I151A / G227S / M228W;
variant R90K / G227S I M228W;
variant I151A / R90K / G227S / M228W.
wherein
R90K refers to R90K in the MPT domain of the amino acid sequence of SEQ ID NO: 1; I151A refers to I151A in the AT domain of the amino acid sequence of SEQ ID NO: 2; G227S refers to G227S in the KS domain of the amino acid sequence of SEQ ID NO: 3; and M228W refers to M228W in the KS domain of the amino acid sequence of SEQ ID NO: 3.

8. The protein according to any one of claims 1 to 7, resulting in elevated overall production of short fatty acids, CoA esters of short fatty acids, ethyl esters of short fatty acids, esters of short fatty acids with other metabolites, and/or enzyme bound short fatty acids (C₆ to C₁₂) compared to the wild type protein(s) or the protein(s) without such amino acid substitution(s).

9. The protein according to any one of claims 1 to 8, wherein the amino acid substitution(s) selected from
variant I151A / G227S / M228W;
variant R90K / G227S / M228W;
wherein
R90K refers to R90K in the MPT domain of the amino acid sequence of SEQ ID NO: 1; I151A refers to I151A in the AT domain of the amino acid sequence of SEQ ID NO: 2; G227S refers to G227S in the KS domain of the amino acid sequence of SEQ ID NO: 3;
M228W refers to M228W in the KS domain of the amino acid sequence of SEQ ID NO: 3; and
L256Y refers to L256Y in the KS domain of the amino acid sequence of SEQ ID NO: 3;
increase(s) the selectivity for the production of C₆ fatty acids, C₆ fatty acid CoA esters, C₆ fatty acid ethyl esters, C₆ fatty acid esters with other metabolites, and/or enzyme bound C₆ fatty acids compared to wild type protein(s) or the protein without such amino acid substitution(s); and/or
increase(s) the selectivity for the production of C₈ fatty acids, C₈ fatty acid CoA esters, C₈ fatty acid ethyl esters, C₈ fatty acid esters with other metabolites, and/or enzyme bound C₈ fatty acids compared to wild type protein(s) or the protein without such amino acid substitution(s); and/or
increase(s) the selectivity for the production of C₁₀ to C₁₂ fatty acids, C₁₀ to C₁₂ fatty acid CoA esters, C₁₀ to C₁₂ fatty acid ethyl esters, C₁₀ to C₁₂ fatty acid esters with other metabolites, and/or enzyme bound C₁₀ to C₁₂ fatty acids compared to wild type protein(s) or the protein without such amino acid substitution(s).

10. A *nucleic acid molecule*, coding for a protein or for a polypeptide domain according to any one of claims 1 to 9,
preferably, further comprising vector nucleic acid sequences, more preferably expression vector sequences, and/or comprising promoter nucleic acid sequences and terminator nucleic acid sequences, and/or comprising other regulatory nucleic acid sequences and/or wherein the nucleic acid molecule preferably comprises dsDNA, ssDNA, cDNA, LNA, PNA, CNA, RNA or mRNA or combinations thereof.

11. A *host cell*, containing a nucleic acid molecule according to claim 10 and preferably expressing said nucleic acid molecule,
wherein said host cell is preferably selected from
- a bacterial cell ,
more preferably a *Corynebacterium*, *Mycobacterium, Escherichia, Nocordia, Bacillus, Clostridium*, *Pseudomonas, Lactobacillus* or *Leuconostoc* cell,
such as *Corynebacterium glutamicum, Escherichia coli, Bacillus subtilis; Clostridium ljungdahlii, Pseudomonas putida; Lactobacillus bifermentans* or *Leuconostoc mesenteroides,*
- a fungus cell,
more preferably a yeast cell,
such as *Saccharomyces* species, *Kluyveromyces* sp., *Hansenula* sp., *Arxula* sp., *Rhodosporidium* sp., *Pichia* sp. or *Yarroowia* sp,
or
- an algae cell,
more preferably a *Chlamydomonas*, *Chlorella*, *Haematococcus*, *Dunaliella, Nannochloropsis, Thalassiosira, Phaeodactylum, Porphyridium* or *Scenedesmus* cell,
such as *Chlamydomonas reinhardtii* or *Haematococcus pluvialis,*
which, more preferably, belongs to the species *Escherichia coli.*

12. The host cell according to claim 11,
which has an elevated overall production of short fatty acids, CoA esters of short fatty acids, ethyl esters of short fatty acids, esters of short fatty acids with other metabolites, and/or enzyme bound short fatty acids (C₆ to C₁₂) compared to a cell not containing a nucleic acid molecule according to claim 10,
or which has an increased yield of C₆ fatty acids, C₆ fatty acid CoA esters, C₆ fatty acid ethyl esters, C₆ fatty acid esters with other metabolites, and/or enzyme bound C₆ fatty acids compared to a cell not containing a nucleic acid molecule according to claim 10,
or which has an increased yield of C₈ fatty acids, C₈ fatty acid CoA esters, C₈ fatty acid ethyl esters, C₈ fatty acid esters with other metabolites, and/or enzyme bound C₈ fatty acids compared to a cell not containing a nucleic acid molecule according to claim 10,
or which has an increased yield of C₁₀ to C₁₂ fatty acids, C₁₀ to C₁₂ fatty acid CoA esters, C₁₀ to C₁₂ fatty acid ethyl esters, C₁₀ to C₁₂ fatty acid esters with other metabolites, and/or enzyme bound C₁₀ to C₁₂ fatty acids compared to a cell not containing a nucleic acid molecule according to claim 10.

13. A *method for the production of short fatty acids,* CoA esters of short fatty acids, short fatty acid ethyl esters, short fatty acid esters with other metabolites, and/or enzyme bound short fatty acids (C₆ to C₁₂), comprising the expression of a nucleic acid molecule according to claim 10, preferably in a host cell according to claim 11 or 12.

14. A *method for the production of biofuels* (such as short alkanes, short alkenes, short alkynes, methyl ketones, short esters or alcohols), flavoring compounds (such as fatty acids esterified with short alcohols) and/or fine chemicals (such as natural compounds), comprising the expression of a nucleic acid molecule according to claim 10, preferably in a host cell according to claim 11 or 12.

15. *Use of a proteins* according to any one of claims 1 to 9, a nucleic acid molecule according to claim 10, or a host cell according to claim 11 or 12, for:
- the production of short fatty acids (C₆ to C₁₂),
- the production of CoA esters of short fatty acids (C₆ to C₁₂),
- the production of ethyl esters of short fatty acids (C₆ to C₁₂),
- the production of short fatty acids (C₆ to C₁₂) esters with other metabolites,
- the production of enzyme bound short fatty acids (C₆ to C₁₂),
- the production of biofuels, such as short alkanes, short alkenes, short alkynes, methyl ketones, short esters and/or alcohols,
- the production of fine chemicals, such as natural compounds where preferably short fatty acids (C₆ to C₁₂) or their derivatives (such as CoA esters, methyl/ethyl esters, esters with other metabolites, enzyme bound fatty acids, alcohols) are used as a building block, or
- the production of flavoring substances, such as esters from short fatty acids (C₆ to C₁₂).


SEQUENCE LISTING

<110>  Johann Wolfgang Goethe-Universität Frankfurt

<120>  Microbiological production of short fatty acids and uses thereof

<130>  u30648EP

<160>  23

<170>  PatentIn version 3.5

<210>  1
<211>  347
<212>  PRT
<213>  Corynebacterium ammoniagenes

<400>  1

Ser Ile Leu Glu Ile Val Arg Asn Asn Pro Pro Leu Val Val Val Asp
1               5                   10                  15

Gly Glu Glu Phe Thr His Pro Asp Gly Thr Leu Phe Leu Thr Gln Phe
            20                  25                  30

Thr Gln Val Ala Met Ala Thr Leu Gly Cys Ala Gln Ile Ala Glu Leu
        35                  40                  45

Ala Glu Ala Gly Val Lys Ser Gln Asp Thr Tyr Phe Ala Gly His Ser
    50                  55                  60

Val Gly Glu Tyr Asn Ala Leu Ala Ala Tyr Ala Gln Val Leu Ser Leu
65                  70                  75                  80

Glu Ala Val Val Glu Ile Val Tyr Ala Arg Gly Leu Thr Met His Arg
                85                  90                  95

Leu Val Asp Arg Asp Ala Glu Gly Asn Ser Asn Tyr Gly Leu Ala Ala
            100                 105                 110

Leu Arg Pro Asn Lys Ile Gly Ile Arg Ala Glu Asp Val Phe Asp Tyr
        115                 120                 125

Val Ala Gln Val Ser Gln Thr Arg Gly Glu Phe Leu Glu Ile Val Asn
    130                 135                 140

Tyr Asn Ile Ala Gly Val Gln Tyr Ala Val Ala Gly Thr Arg Ala Gly
145                 150                 155                 160

Leu Ala Ala Leu Ala Ala Asp Ala Glu Ser Arg Ala Pro Gly Met Arg
                165                 170                 175

Ser Phe Ile Met Ile Pro Gly Ile Asp Val Pro Phe His Ser Ser His
            180                 185                 190

Leu Leu Gly Gly Val Asp Asn Phe Arg Thr His Leu Asp Met Leu Ile
        195                 200                 205

Pro Asp Asp Val Asp Leu Asp Ile Leu Arg Gly Arg Tyr Ile Pro Asn
    210                 215                 220

Leu Val Ala Arg Pro Phe Glu Leu Thr Arg Glu Phe Val Glu Ala Met
225                 230                 235                 240

Ala Asp Val Val Asp Ser Thr Tyr Val Asn Asp Ile Leu Ala Asp Phe
                245                 250                 255

Asp Lys Ala Thr Asp Asp Pro Ala Lys Met Gly Arg Thr Leu Leu Ile
            260                 265                 270

Glu Leu Leu Ala Trp Gln Phe Ala Ser Pro Val Arg Trp Ile Glu Thr
        275                 280                 285

Gln Asp Leu Leu Leu Ser Ala Pro Ser Val Ser Ala Gly Ala Gly Thr
    290                 295                 300

Ala Gly Leu Gly Val Glu Arg Phe Val Glu Ile Gly Val Gly Ser Ser
305                 310                 315                 320

Pro Thr Leu Ala Asn Met Leu Gly Gln Thr Leu Arg Leu Pro Gln Tyr
                325                 330                 335

Ala Gly Asn Pro Ile Glu Val Leu Asn Val Glu
            340                 345

<210>  2
<211>  349
<212>  PRT
<213>  Corynebacterium ammoniagenes

<400>  2

Met Thr Ile Gly Ile Ser Asn His Arg Leu Val Asn Arg Leu Glu Ser
1               5                   10                  15

Glu Lys Phe Ala Leu Ser Phe Ser Gly Gln Gly Phe Ser Trp Leu Pro
            20                  25                  30

Thr Leu Arg Thr Val Ile Ala Gly Gly Asn Gly Ala Gln Val Ala Gly
        35                  40                  45

Ala Val Asp Ala Ala Gly Lys Leu Leu Glu Pro Val Ala Asp Glu Leu
    50                  55                  60

Val Ala Ala Leu Pro His Gly Phe Asp Pro Val Glu Trp Ala Gly Asn
65                  70                  75                  80

Ala Glu Asp Pro Ala Trp Asp Leu Ser Asp Ser Ser Ile Ser Thr Pro
                85                  90                  95

Gly Ile Val Val Ala Gln Tyr Gly Leu Ile Glu Ser Leu Lys Ala Gln
            100                 105                 110

Gly Leu Asp Pro Ala Asp Ala Val Ala His Ile Gly His Ser Gln Gly
        115                 120                 125

Ala Leu Ala Thr Tyr Ile Ser Ser Gly Arg Ala Gln Ala Ala Glu Val
    130                 135                 140

Ile Ala Leu Ala Gln Leu Ile Gly Val Ala Ile Ser Lys Thr Ala Arg
145                 150                 155                 160

Ala Thr Gly Leu Ile Arg Ile Ala Ala Gly Ser Pro Met Val Ser Ile
                165                 170                 175

Ala Gly Ile Ser Arg Glu Gln Leu Ala Lys Val Leu Ala Lys Thr Gly
            180                 185                 190

Val Asp Ser Val Ile Gly Leu Gln Asn Asn His Asn Thr Phe Val Val
        195                 200                 205

Thr Gly Ser Pro Glu Asp Asn Asn Lys Val Ile Glu Ala Ile Glu Lys
    210                 215                 220

Leu Ala Ala Lys Asp Ala Lys Ala Ile Glu Asn Lys Glu Arg Gly Gly
225                 230                 235                 240

Asn Pro Phe Ala Pro Arg Ile Asn Ala Leu Pro Val Gln Ser Gly Tyr
                245                 250                 255

His His Pro Lys Met Ser Ala Ala Val Glu Met Val Thr Ser Trp Ala
            260                 265                 270

Glu Arg Ile Gly Leu Asp Lys Asp Leu Ala His Asp Gly Ala Glu Ala
        275                 280                 285

Val Leu Thr Glu Ile Val Asn Trp Pro Ala Thr Val Gln Ser Ala Val
    290                 295                 300

Asp Ala Gly Val Thr Trp Ile Leu Glu Cys Gly Pro Glu Lys Gly Met
305                 310                 315                 320

Ala Ala Leu Thr Lys Ala Val Val Ala Gly Gln Gly Ile Gly Val Gln
                325                 330                 335

Val Val Ser Thr Asp Glu Gly Gln Ala Ala Leu Phe Asp
            340                 345

<210>  3
<211>  558
<212>  PRT
<213>  Corynebacterium ammoniagenes

<400>  3

Leu Asp Asp Met Val Val Ile Val Gly Ala Gly Glu Ile Gly Pro Val
1               5                   10                  15

Gly Ser Ser Arg Thr Arg Phe Glu Val Glu Met Thr Gly Asp Leu Thr
            20                  25                  30

Ala Ala Gly Val Val Glu Leu Ala Trp Thr Thr Gly Leu Ile Ala Trp
        35                  40                  45

Asp Asp Ala Ser Gly Ala Trp Phe Asp Ala Glu Gly Glu Glu Ile Ala
    50                  55                  60

Glu Glu Asp Ile Tyr Asp Arg Phe His Asp Glu Val Leu Ala Asn Val
65                  70                  75                  80

Gly Val Arg Lys Tyr His Asp Asp Tyr Gly Pro Lys Met Pro Met Val
                85                  90                  95

Asp Asn Leu Ala Pro Glu Leu Thr Thr Ile Tyr Leu Glu Arg Asp Met
            100                 105                 110

Thr Phe Ala Val Ala Asp Glu Glu Thr Ala Arg Thr Phe Val Ala Asp
        115                 120                 125

Val Glu Gly Ala Thr Ala His Phe Asp Gly Glu Glu Trp Gln Val Thr
    130                 135                 140

Arg Pro Ala Gly Ala Glu Val Arg Val Pro Arg Arg Val Ala Met Thr
145                 150                 155                 160

Arg Phe Val Gly Gly Gln Ile Pro Gln Gly Phe Asp Pro Gly Val Tyr
                165                 170                 175

Gly Ile Pro Ala Asp Met Ile Asp Asn Leu Asp Arg Val Ala Leu Trp
            180                 185                 190

Asn Leu Val Cys Thr Val Asp Ala Phe Leu Ala Ala Gly Phe Ser Pro
        195                 200                 205

Ala Glu Leu Leu Ala His Val His Pro Ala Arg Val Ser Ser Thr Gln
    210                 215                 220

Gly Thr Gly Met Gly Gly Met Gln Ser Met Arg Ser Leu Tyr Leu Asp
225                 230                 235                 240

Lys Leu Leu Ala Gln Pro Arg Pro Asn Asp Ile Leu Gln Glu Ala Leu
                245                 250                 255

Pro Asn Val Val Ala Ala His Val Met Gln Ser Tyr Val Gly Gly Tyr
            260                 265                 270

Gly Gln Met Val His Pro Val Ala Ala Cys Ala Thr Ala Ala Val Ser
        275                 280                 285

Val Glu Glu Gly Met Asp Lys Leu Arg Leu Arg Lys Ala Asp Phe Val
    290                 295                 300

Val Ala Gly Gly Ile Asp Asp Leu Ser Ile Glu Gly Ile Thr Gly Phe
305                 310                 315                 320

Gly Asp Met Ala Ala Thr Ala Asp Ser Gly Glu Met Glu Ala Lys Gly
                325                 330                 335

Ile Asp His Lys Tyr Phe Ser Arg Ala Asn Asp Arg Arg Arg Gly Gly
            340                 345                 350

Phe Ile Glu Ser Glu Gly Gly Gly Thr Ile Leu Leu Ala Arg Gly Ser
        355                 360                 365

Leu Ala Ala Glu Leu Gly Leu Pro Val Leu Gly Val Val Gly Phe Ala
    370                 375                 380

Glu Ser Phe Ala Asp Gly Ala His Thr Ser Ile Pro Ala Pro Gly Leu
385                 390                 395                 400

Gly Ala Leu Ser Ala Ala Arg Gly Gly Ala Asp Ser Arg Leu Ala Gln
                405                 410                 415

Gly Leu Ala Glu Leu Gly Val Ser Ala Asn Glu Ile Ala Val Ile Ser
            420                 425                 430

Lys His Asp Thr Ser Thr Asn Ala Asn Asp Pro Asn Glu Ser Asp Leu
        435                 440                 445

His Glu Arg Ile Ala Ala Ser Met Gly Arg Asp Ala Gly Asn Pro Leu
    450                 455                 460

Phe Ile Ile Ser Gln Lys Thr Leu Thr Gly His Ala Lys Gly Gly Ala
465                 470                 475                 480

Ala Ala Phe Gln Leu Val Gly Leu Thr Gln Val Leu Arg Asn Gly Val
                485                 490                 495

Leu Pro Pro Asn Arg Ser Leu Asp Cys Val Asp Pro Ala Leu Arg Gln
            500                 505                 510

His Thr His Leu Thr Trp Leu Arg Glu Gln Leu Asp Leu Arg Ala Thr
        515                 520                 525

Pro Pro Lys Ala Gly Leu Val Thr Ser Leu Gly Phe Gly His Val Ser
    530                 535                 540

Ala Leu Val Ala Val Val His Pro Gly Ala Phe Val Glu Ala
545                 550                 555

<210>  4
<211>  1041
<212>  DNA
<213>  Corynebacterium ammoniagenes

<400>  4
tccattttgg aaattgtacg caacaaccca ccgctggtcg ttgtcgacgg cgaagagttc      60

acccacccag atggcactct attcttaacc cagttcacgc aggtcgcgat ggcgacattg     120

ggctgcgccc aaatcgcgga gctggcagaa gccggcgtga aatcccagga cacttacttc     180

gccggacact ccgtgggcga atacaacgcc ctagctgcct acgcacaggt gctgtctttg     240

gaagccgttg tagaaatcgt ctacgccaag ggtttgacca tgcaccgcct ggtcgaccgt     300

gacgcagaag gcaactccaa ctacggcctg gcagcgctgc gcccgaataa gattggcatc     360

cgcgccgagg acgtctttga ctacgtcgcg caggtctcgc agacccgcgg cgaattccta     420

gaaatcgtca actacaacat cgccggtgtg cagtacgccg tcgccggcac gcgagcaggg     480

ttggcagcat tggctgccga tgccgagtcg cgcgcgccgg gcatgcgctc atttatcatg     540

atcccgggca tcgacgtgcc atttcactct tcgcacctgt tgggcggcgt cgataacttc     600

cgcacgcacc tggacatgct cattccagac gacgtggatt tggatattct gcgcggtcgc     660

tacatcccta acctggtggc tcgcccgttt gagctcaccc gcgaattcgt cgaagccatg     720

gccgatgtcg tcgattcgac gtacgtcaac gacatcctcg ccgacttcga taaggccacg 780

gacgacccgg ccaagatggg ccgcaccctg ctcattgagc tcttagcgtg gcaatttgcc 840

tcacctgtgc gctggatcga aacccaagac ttgctgctgt ctgccccaag cgtcagcgcc 900

ggagccggca ccgcaggtct aggcgtggag cgctttgtag aaatcggcgt gggttcttcc 960

ccgacgcttg cgaatatgct cggccaaacc ctgcgcttgc cgcaatacgc cggcaatccc 1020

attgaggttc tcaacgtgga a 1041

<210> 5
<211> 1047
<212> DNA
<213> Corynebacterium ammoniagenes

<400> 5
gtgactattg gcatctctaa ccaccgcctg gtgaaccgac tcgagtctga aaaatttgct 60

ctcagctttt ccggacaagg ttttagctgg ttgcccacct tgcgcacggt aattgccggt 120

ggcaatggcg cgcaggtagc cggagcagtc gatgcagcgg gcaagttgct ggagccagta 180

gcagatgagc tcgttgctgc actgccacac ggcttcgatc cggtggagtg ggctggcaac 240

gccgaggacc cagcgtggga tttgtcagat tcgtcgattt ctaccccggg catcgtggtc 300

gcccagtacg gcctgattga gtcattgaaa gcccagggat tagatcctgc ggatgcagtc 360

gcacacattg gccattccca aggcgcgctt gctacttata tctcctccgg gcgcgcgcag 420

gcagcagagg ttattgcgct ggcgcagctc gccggcgtcg ctatttctaa gaccgcacgc 480

gccacgggac tcatccgtat cgctgcgggt tcaccgatgg tctccatcgc cggcatttcc 540

cgcgagcagc tggccaaggt gctggctaaa accggtgtcg atagcgttat tggcctgcaa 600

aataaccaca acactttcgt tgttaccggc agcccggaag ataataataa ggtcattgag 660

gcgattgaaa aattagccgc caaagacgcg aaagcgattg aaaataaaga acgcggcggc 720

aacccctttg ccccgcgtat taatgctttg ccggtgcaat ctggctacca ccacccaaag 780

atgtccgcgg cagtagagat ggtcaccagc tgggcagagc gcatcgggct ggataaagac 840

ttggctcacg atggcgcaga ggcagtcttg acggagatcg tgaactggcc tgccacggtg 900

cagtccgctg tcgatgcggg cgtgacgtgg attttggaat gcggtccgga aaagggcatg 960

gctgccttga ccaaggcggt cgtcgccggt caaggcattg gggtgcaggt tgtctccacg 1020

gacgaaggcc aagcagcgct tttcgat 1047

<210> 6
<211> 1674
<212> DNA
<213> Corynebacterium ammoniagenes

<400> 6

ctcgatgaca tggtggttat cgtcggtgcc ggcgaaattg ggccggtggg atcgtctcgg 60

acccgtttcg aggtggagat gaccggtgac cttaccgccg ctggtgttgt cgaattggcg 120

tggactactg gcctgattgc ctgggatgat gcctcgggtg cgtggtttga tgccgagggc 180

gaggaaatcg ccgaggaaga tatctacgac cgcttccacg atgaagtcct agccaacgtc 240

ggcgtacgca agtaccacga tgactacggc ccgaagatgc cgatggtgga taacttggct 300

ccggagctga ccaccatcta cctcgaacgc gatatgacct ttgctgttgc ggatgaagaa 360

accgcgcgca cgtttgttgc cgacgtggaa ggtgccaccg cgcactttga cggcgaggaa 420

tggcaagtca cccgcccagc tggggccgag gtgcgcgtgc cgcggcgcgt ggcgatgacg 480

cgctttgtcg gcgggcagat tccgcaaggg tttgaccccg gtgtctacgg tattccggcc 540

gatatgattg acaacctcga ccgggtggcc ttgtggaacc tggtgtgcac ggtggatgct 600

ttcttggcgg ctgggtttag ccccgcggaa ttgctggcgc atgtgcaccc ggcgcgcgtg 660

agctcgaccc agggcacgag ttggggcggc atgcagtcga tgcgctcgct gtacctggac 720

aagctgctgg cgcagccgcg cccgaatgac atcttgcagg aagcactgcc gaatgtcgtg 780

gcagcgcacg tcatgcagtc ttatgtcggc ggttatggcc agatggttca cccggttgcc 840

gcgtgtgcga ccgcggctgt ctctgtcgaa gagggcatgg ataaactgcg tttgcgcaag 900

gctgatttcg tggtcgccgg cggtattgat gacctgtcga ttgaaggcat tacgggcttc 960

ggcgatatgg cggccacggc ggattccggc gagatggaag ccaagggcat tgaccacaag 1020

tacttctcgc gtgccaatga ccgtcgtcgt ggcgggttca ttgagtcgga aggcggcggc 1080

accatcttgc tggcgcgcgg tagcttggct gcggaattgg gcctgccagt cttgggcgta 1140

gttggcttcg cggagtcttt cgccgacggt gcgcacacct ccattccagc gcctggtctg 1200

ggtgcgcttt ctgccgcgcg tggtggggca gattcccgtc tagcgcaggg attggccgag 1260

cttggggtgt ctgcgaatga aatcgcggtc atttccaagc acgacacgtc tacgaatgcc 1320

aatgacccga atgagtctga cctgcatgag cgaatcgcag cgtcaatggg ccgcgatgcg 1380

ggcaacccgt tgtttatcat ctcgcaaaag accctgaccg gtcacgccaa gggcggtgcc 1440

gcagccttcc agctggttgg tctcacccag gttctgcgca acggtgtatt gccaccgaac 1500

cgctcgttgg actgtgtgga tccggcgttg cgccagcaca ctcatttgac ctggctgcgc 1560

gagcagctgg atttgcgtgc aaccccacca aaggccgggc tggttacatc cttgggcttt 1620

ggtcacgtct ccgcgctggt tgctgtggtt caccctggcg cattcgtgga agct 1674

<210> 7
<211> 3016
<212> PRT
<213> Corynebacterium ammoniagenes

<400> 7

Met Thr Ile Gly Ile Ser Asn His Arg Leu Val Asn Arg Leu Glu Ser
1               5                   10                  15

Glu Lys Phe Ala Leu Ser Phe Ser Gly Gln Gly Phe Ser Trp Leu Pro
            20                  25                  30

Thr Leu Arg Thr Val Ile Ala Gly Gly Asn Gly Ala Gln Val Ala Gly
        35                  40                  45

Ala Val Asp Ala Ala Gly Lys Leu Leu Glu Pro Val Ala Asp Glu Leu
    50                  55                  60

Val Ala Ala Leu Pro His Gly Phe Asp Pro Val Glu Trp Ala Gly Asn
65                  70                  75                  80

Ala Glu Asp Pro Ala Trp Asp Leu Ser Asp Ser Ser Ile Ser Thr Pro
                85                  90                  95

Gly Ile Val Val Ala Gln Tyr Gly Leu Ile Glu Ser Leu Lys Ala Gln
            100                 105                 110

Gly Leu Asp Pro Ala Asp Ala Val Ala His Ile Gly His Ser Gln Gly
        115                 120                 125

Ala Leu Ala Thr Tyr Ile Ser Ser Gly Arg Ala Gln Ala Ala Glu Val
    130                 135                 140

Ile Ala Leu Ala Gln Leu Ile Gly Val Ala Ile Ser Lys Thr Ala Arg
145                 150                 155                 160

Ala Thr Gly Leu Ile Arg Ile Ala Ala Gly Ser Pro Met Val Ser Ile
                165                 170                 175

Ala Gly Ile Ser Arg Glu Gln Leu Ala Lys Val Leu Ala Lys Thr Gly
            180                 185                 190

Val Asp Ser Val Ile Gly Leu Gln Asn Asn His Asn Thr Phe Val Val
        195                 200                 205

Thr Gly Ser Pro Glu Asp Asn Asn Lys Val Ile Glu Ala Ile Glu Lys
    210                 215                 220

Leu Ala Ala Lys Asp Ala Lys Ala Ile Glu Asn Lys Glu Arg Gly Gly
225                 230                 235                 240

Asn Pro Phe Ala Pro Arg Ile Asn Ala Leu Pro Val Gln Ser Gly Tyr
                245                 250                 255

His His Pro Lys Met Ser Ala Ala Val Glu Met Val Thr Ser Trp Ala
            260                 265                 270

Glu Arg Ile Gly Leu Asp Lys Asp Leu Ala His Asp Gly Ala Glu Ala
        275                 280                 285

Val Leu Thr Glu Ile Val Asn Trp Pro Ala Thr Val Gln Ser Ala Val
    290                 295                 300

Asp Ala Gly Val Thr Trp Ile Leu Glu Cys Gly Pro Glu Lys Gly Met
305                 310                 315                 320

Ala Ala Leu Thr Lys Ala Val Val Ala Gly Gln Gly Ile Gly Val Gln
                325                 330                 335

Val Val Ser Thr Asp Glu Gly Gln Ala Ala Leu Phe Asp Val Gly Gln
            340                 345                 350

Ala Pro Glu Leu Pro Val Asn Phe Ala Gln Phe Ala Pro Lys Val Phe
        355                 360                 365

Asp Asp Lys Gly Lys Thr Arg Leu Ser Thr Arg Phe Thr Glu Leu Thr
    370                 375                 380

Gly Tyr Ser Pro Val Val Leu Ala Gly Met Thr Pro Ser Thr Val Asp
385                 390                 395                 400

Pro Ala Ile Val Ala Ala Ala Ala Asn Ala Gly Phe Trp Ala Glu Leu
                405                 410                 415

Ala Gly Gly Gly Gln Val Thr Asp Ala Ile Leu Asn Asp Ser Leu Glu
            420                 425                 430

Arg Leu Glu Asp Met Leu Asn Pro Gly Ile Asn Ala Gln Phe Asn Ala
        435                 440                 445

Met Tyr Leu Ser Pro Lys Gln Trp Arg Ala Gln Ile Glu Gly Arg Arg
    450                 455                 460

Leu Ile Pro Arg Ala Arg Ala Asn Gly Ala Ser Ile Asn Gly Val Ile
465                 470                 475                 480

Cys Ser Ala Gly Ile Pro Pro His Glu Glu Ala Ile Ala Leu Val Arg
                485                 490                 495

Gln Leu Gln Glu Asp Asn Ile Pro Trp Val Ala Phe Lys Pro Gly Ala

500                 505                 510

Val Arg His Val His Gln Val Leu Ala Ile Ala Asp Asp Leu Pro Asp
        515                 520                 525

Thr Thr Val Ile Met Gln Val Glu Gly Gly Lys Ala Gly Gly His His
    530                 535                 540

Ser Trp Glu Asp Leu Asp Asp Leu Leu Thr Glu Thr Tyr Ala Asp Ile
545                 550                 555                 560

Arg Glu Arg Asp Asn Val Val Leu Met Ala Ala Gly Gly Ile Gly Ala
                565                 570                 575

Pro Glu Arg Gly Ala Gln Tyr Leu Thr Gly Glu Trp Ser Lys Val Tyr
            580                 585                 590

Gly Leu Pro Ala Met Pro Val Asp Ala Ile Met Ile Gly Thr Ala Ala
        595                 600                 605

Met Ala Thr Lys Glu Ser Thr Ala Ser Glu Ser Val Lys Gln Ala Leu
    610                 615                 620

Val Ala Thr Gln Gly Leu Glu Asp Ile Pro Gly Gly Gly Trp Val Pro
625                 630                 635                 640

Ala Gly Gly Ala Arg Asp Gly Ile Ala Ser Gly Arg Ser Gln Leu Gly
                645                 650                 655

Ala Asp Ile His Glu Ile Asp Asn Thr Phe Ala Lys Ala Gly Arg Leu
            660                 665                 670

Leu Asp Glu Val Ala Gly Asp Ala Asp Ala Val Ala Ala Arg Arg Glu
        675                 680                 685

Glu Ile Ile Ala Val Ile Ala Gly Thr Ala Lys Pro Tyr Phe Gly Asp
    690                 695                 700

Ile Asn Ala Met Thr Tyr Ala Glu Trp Leu Ala Arg Tyr Val Glu Val
705                 710                 715                 720

Ser Tyr Met Gly Ser Trp Val Asp Phe Ser Trp Ala Arg Arg Phe Glu
                725                 730                 735

Gln Met Ile Ala Arg Ala Glu Ala Arg Leu Thr Glu Ala Asp His Gly
            740                 745                 750

Glu Phe Thr Pro Leu Val Thr Val Asp Ala Glu His Pro Glu Arg Ala
        755                 760                 765

Ile Ala Asp Val Val Ala Ala Tyr Pro Ala Ala Glu Lys His Thr Val
    770                 775                 780

Ile Ala Ser Asp Glu Ala Trp Phe Val Asp Leu Leu Arg Gly Asn Gly
785                 790                 795                 800

Lys Pro Pro Ala Phe Val Pro Val Ile Asp Ser Asp Val Arg Arg Trp
                805                 810                 815

Trp Arg Gln Asp Ser Leu Trp Gln Ala His Asp Glu Arg Tyr Thr Ala
            820                 825                 830

Glu Gln Val Gly Ile Ile Pro Gly Thr Thr Ala Val Ala Gly Ile Thr
        835                 840                 845

Lys Ala Asn Glu Pro Val Ala Glu Leu Leu Ala Arg Phe Glu Ala Ala
    850                 855                 860

Ala Val Glu Gln Ala Leu Gln Ser Glu Asp Thr Glu Lys Ala Glu Leu
865                 870                 875                 880

Ala Asn Ser Ala Leu Glu Arg Val Leu Ala Ala Pro Ser Val Glu Trp
                885                 890                 895

Ala Gly Arg Gln Arg Pro Asn Pro Val Ala Gln Val Ser Glu Ser Trp
            900                 905                 910

Thr Val Asp Ser Gln Ala Lys Thr Ala Ser Thr Glu Gly Ala Glu Leu
        915                 920                 925

Val Thr Ile Asp Glu His Thr Val Glu Leu Ser Val Ala Leu Thr Gly
    930                 935                 940

Ser Val Pro Gly Ala Thr Leu Gln Leu Gln Ile Thr Val Pro Ala Asp
945                 950                 955                 960

Ala Pro Leu Ser Ala Thr Pro Ile Ile Thr Ala Gln Ala Ala Glu Glu
                965                 970                 975

Ser Met Ala Ala Leu Ala Ala Val Ala Ala Gly Gly Thr Leu Pro Glu
            980                 985                 990

Val Thr Asp Gly Thr Ala Val Trp Lys Thr Thr Leu Asp Ser Ala Ala
        995                 1000                1005

Leu Ser Asp Tyr Ala Gly Val Thr Ser Gly Tyr Leu Ala Ser Ala
    1010                1015                1020

Asp Thr Asn Ser Ala Ile Ala Pro Asp Val Ile Val Gly Arg Ala
    1025                1030                1035

Trp Pro Ala Val Phe Ala Val Ile Ala Asn Ser Val Ile Pro Gly
    1040                1045                1050

Ser Asp Ser Ala Leu Val Val Glu Gly Leu Leu Asn Leu Val His
    1055                1060                1065

Leu Glu His Asp Val Arg Leu His Gln Ala Leu Pro Thr Glu Ala
    1070                1075                1080

Val Asp Leu Thr Val Thr Ala Arg Gly Gly Glu Val Arg Asp Thr
    1085                1090                1095

Ala Leu Gly Arg Val Val Glu Val Ala Val Asp Ile Ala Ile Ala
    1100                1105                1110

Glu Ala Glu Asp Thr Val Leu Ala Thr Leu Lys Glu Arg Phe Ala
    1115                1120                1125

Ile Val Gly Arg Thr Gly Glu Gln Glu Leu Glu Pro Ala Thr Ser
    1130                1135                1140

Thr Ala Glu Ile Thr Asn Thr Pro Ala Ser Phe Arg His Gln Leu
    1145                1150                1155

Arg Val Thr Ala Pro Ala Ser Met His Pro Phe Ala Val Val Thr
    1160                1165                1170

Gly Asp Arg Asn Pro Ile His Val Ser Ala Ala Ala Ala Arg Leu
    1175                1180                1185

Ala Gly Leu Glu Asn Gly Val Ile Val His Gly Met Trp Thr Ser
    1190                1195                1200

Ala Ile Gly Gln Leu Ala Ala Gly Phe Asp Gly Leu Glu Leu Ala
    1205                1210                1215

Glu Trp Ser Ala Asn Met Leu Ser Pro Val Leu Pro Gly Ala Glu
    1220                1225                1230

Val Thr Phe Ile Val Glu Arg Thr Gly Val Asp Ser Arg Pro Gly
    1235                1240                1245

Arg Gly Glu Val Arg Thr Val Thr Ala Ser Val Gly Asp Asp Val
    1250                1255                1260

Val Leu Gln Ala Thr Ala Thr Met Ala Ala Pro Arg Thr Phe Tyr
    1265                1270                1275

Gly Phe Pro Gly Gln Gly Ile Gln Ser Gln Gly Met Gly Met Glu
    1280                1285                1290

Ser Tyr Ala Ser Ser Ser Ala Ala Arg Glu Val Trp Glu Arg Ala
    1295                1300                1305

Asp Ala His Thr Arg Glu Lys Leu Gly Phe Ser Ile Leu Glu Ile
    1310                1315                1320

Val Arg Asn Asn Pro Pro Leu Val Val Val Asp Gly Glu Glu Phe
    1325                1330                1335

Thr His Pro Asp Gly Thr Leu Phe Leu Thr Gln Phe Thr Gln Val
    1340                1345                1350

Ala Met Ala Thr Leu Gly Cys Ala Gln Ile Ala Glu Leu Ala Glu
    1355                1360                1365

Ala Gly Val Lys Ser Gln Asp Thr Tyr Phe Ala Gly His Ser Val
    1370                1375                1380

Gly Glu Tyr Asn Ala Leu Ala Ala Tyr Ala Gln Val Leu Ser Leu
    1385                1390                1395

Glu Ala Val Val Glu Ile Val Tyr Ala Arg Gly Leu Thr Met His
    1400                1405                1410

Arg Leu Val Asp Arg Asp Ala Glu Gly Asn Ser Asn Tyr Gly Leu
    1415                1420                1425

Ala Ala Leu Arg Pro Asn Lys Ile Gly Ile Arg Ala Glu Asp Val
    1430                1435                1440

Phe Asp Tyr Val Ala Gln Val Ser Gln Thr Arg Gly Glu Phe Leu
    1445                1450                1455

Glu Ile Val Asn Tyr Asn Ile Ala Gly Val Gln Tyr Ala Val Ala
    1460                1465                1470

Gly Thr Arg Ala Gly Leu Ala Ala Leu Ala Ala Asp Ala Glu Ser

1475                1480                1485

Arg Ala Pro Gly Met Arg Ser Phe Ile Met Ile Pro Gly Ile Asp
    1490                1495                1500

Val Pro Phe His Ser Ser His Leu Leu Gly Gly Val Asp Asn Phe
    1505                1510                1515

Arg Thr His Leu Asp Met Leu Ile Pro Asp Asp Val Asp Leu Asp
    1520                1525                1530

Ile Leu Arg Gly Arg Tyr Ile Pro Asn Leu Val Ala Arg Pro Phe
    1535                1540                1545

Glu Leu Thr Arg Glu Phe Val Glu Ala Met Ala Asp Val Val Asp
    1550                1555                1560

Ser Thr Tyr Val Asn Asp Ile Leu Ala Asp Phe Asp Lys Ala Thr
    1565                1570                1575

Asp Asp Pro Ala Lys Met Gly Arg Thr Leu Leu Ile Glu Leu Leu
    1580                1585                1590

Ala Trp Gln Phe Ala Ser Pro Val Arg Trp Ile Glu Thr Gln Asp
    1595                1600                1605

Leu Leu Leu Ser Ala Pro Ser Val Ser Ala Gly Ala Gly Thr Ala
    1610                1615                1620

Gly Leu Gly Val Glu Arg Phe Val Glu Ile Gly Val Gly Ser Ser
    1625                1630                1635

Pro Thr Leu Ala Asn Met Leu Gly Gln Thr Leu Arg Leu Pro Gln
    1640                1645                1650

Tyr Ala Gly Asn Pro Ile Glu Val Leu Asn Val Glu Arg Asp Arg
    1655                1660                1665

Ala Thr Val Phe Ala Glu Asp Ala Leu Glu Arg Pro Val Glu Asp
    1670                1675                1680

Ser Ser Glu Ser Ser Thr Gly Val Ala Glu Ala Ala Thr Ala Thr
    1685                1690                1695

Ala Glu Glu Val Ser Gln Ala Asn Ala Ala Pro Ala Ala Ala Pro
    1700                1705                1710

Glu Pro Val Val Ala Gln Pro Ala Thr Pro Ala Pro Ala Ala Gly
    1715                1720                1725

Gly Thr Thr Pro Asp Asp Lys Ala Phe Ser Ala Ala Asp Ala Thr
    1730                1735                1740

Glu Met Leu Ile Ala Ile Trp Thr Lys Val Arg Pro Asp Gln Met
    1745                1750                1755

Gly Ala Ala Asp Thr Ile Glu Leu Leu Val Glu Gly Val Ser Ser
    1760                1765                1770

Arg Arg Asn Gln Leu Leu Leu Asp Leu Gly Val Glu Phe Gly Leu
    1775                1780                1785

Gly Ala Ile Glu Gly Ala Ala Asp Ala Glu Ile Ser Ala Leu Lys
    1790                1795                1800

Glu Gln Val Ser Gly Met Ala Lys Gly Tyr Lys Ala Phe Gly Pro
    1805                1810                1815

Val Leu Ser Ala Gln Val Ala Glu Ser Leu Arg Arg Leu Thr Gly
    1820                1825                1830

Pro Ala Gly Lys Lys Pro Ala Tyr Ile Ala Gln Arg Val Ala Asp
    1835                1840                1845

Thr Trp Gly Leu Gly Ala Gly Trp Val Asp Arg Val Thr Ala Glu
    1850                1855                1860

Leu Val Leu Gly Ala Arg Glu Gly Ala Ser Leu Arg Gly Gly Asp
    1865                1870                1875

Leu Ala Leu Leu Ala Pro Ala Asn Pro Ala Thr Thr Ala Glu Leu
    1880                1885                1890

Asp Ala Leu Ile Asp Ala Ala Val Asp Ala Ala Gly Ala Lys Val
    1895                1900                1905

Gly Ile Val Ile Ser Lys Pro Ser Ala Ser Gly Ala Ala Gln Gly
    1910                1915                1920

Gly Val Val Asp Ser Ala Ala Leu Asp Ala Tyr Ser Glu Lys Met
    1925                1930                1935

Asn Ser Ala Leu Ala Asn Thr Ala Arg Thr Leu Leu Ser Gln Leu
    1940                1945                1950

Gly Gln Gln Ala Pro Thr Thr Glu Phe Ser Glu Ala Asp Asp Asn
    1955                1960                1965

Ala Ala Val Val Asp Leu Val Thr Ser Glu Leu Gly Ala Asp Trp
    1970                1975                1980

Pro Arg Leu Val Ser Pro Ser Phe Asp Ala Asp Lys Thr Val Gln
    1985                1990                1995

Leu Asp Asp Arg Trp Ala Ser Ala Arg Glu Asp Leu Val Arg Leu
    2000                2005                2010

Ser Leu Gly Glu Ile Glu Asp Leu Asp Ile Thr Gly Ala Gly Glu
    2015                2020                2025

Glu Ala Ala Ala Met Ala Thr Tyr Leu Gly Leu Asp Glu Gln Ala
    2030                2035                2040

Ala Gln Ala Arg Val Ala Ala Ser Thr Leu Asp His Asp Gly Glu
    2045                2050                2055

Val Ala Val Val Thr Gly Gly Ser Pro Gly Ser Ile Ala Ser Glu
    2060                2065                2070

Ile Val Ala Asn Leu Leu Arg Glu Gly Ala Thr Val Ile Ala Thr
    2075                2080                2085

Thr Ser Arg Leu Gly His Asp Arg Leu Glu Phe Tyr Lys Asn Leu
    2090                2095                2100

Tyr Arg Thr Ser Ala Arg Gly Asn Ala Gln Leu Trp Ile Val Pro
    2105                2110                2115

Ala Asn Leu Asn Ser Phe Ala Asp Leu Asp Ala Leu Ile Asp Trp
    2120                2125                2130

Ile Gly Thr Glu Gln Thr Val Thr Val Gly Gly Ser Ser Lys Leu
    2135                2140                2145

Val Lys Pro Ala Leu Val Pro Thr Leu Leu Phe Pro Phe Ala Ala
    2150                2155                2160

Pro Arg Val Gln Gly Thr Leu Ala Asp Ala Gly Ala Pro Ala Glu
    2165                2170                2175

Ala Gln Met Arg Leu Leu Leu Trp Ser Val Glu Lys Leu Ile Ala
    2180                2185                2190

Gly Leu Ser Gln Leu Gly Thr Gly Thr His Val Gly Gln Arg Leu
    2195                2200                2205

His Ile Val Leu Pro Gly Ser Pro Asn Arg Gly Arg Phe Gly Gly
    2210                2215                2220

Asp Gly Ala Tyr Gly Glu Ser Lys Ala Ala Leu Asp Ala Leu Val
    2225                2230                2235

Thr Arg Trp His Ala Glu Pro Val Trp Gly Ala Arg Thr Ser Ile
    2240                2245                2250

Val His Ala His Ile Gly Trp Val Arg Gly Thr Gly Leu Met Gly
    2255                2260                2265

Gly Asn Asp Pro Leu Val Asp Ala Val Glu Ala Lys Gly Val Arg
    2270                2275                2280

Thr Phe Ser Thr Glu Glu Met Ala Ser Gln Leu Val Gly Glu Gly
    2285                2290                2295

Ala Ser Thr Ser Lys Arg Asp Ala Ala Ala His Ala Pro Val Thr
    2300                2305                2310

Leu Asp Leu Thr Gly Gly Leu Gly Glu Ala Asp Ile Asn Leu Pro
    2315                2320                2325

Glu Leu Ala Arg Ser Val Ser Val Gln Pro Val Ala Glu Asp Val
    2330                2335                2340

Glu Glu Val Ala Ser Leu Arg Ala Leu Pro Asn Ile Arg Glu Arg
    2345                2350                2355

Ile Glu Trp Thr Thr Pro Asp Phe Ala Gly Val Asn Gln Lys Leu
    2360                2365                2370

Asp Asp Met Val Val Ile Val Gly Ala Gly Glu Ile Gly Pro Val
    2375                2380                2385

Gly Ser Ser Arg Thr Arg Phe Glu Val Glu Met Thr Gly Asp Leu
    2390                2395                2400

Thr Ala Ala Gly Val Val Glu Leu Ala Trp Thr Thr Gly Leu Ile
    2405                2410                2415

Ala Trp Asp Asp Ala Ser Gly Ala Trp Phe Asp Ala Glu Gly Glu

2420                    2425                    2430

Glu Ile Ala Glu Glu Asp Ile Tyr Asp Arg Phe His Asp Glu Val
    2435                    2440                    2445

Leu Ala Asn Val Gly Val Arg Lys Tyr His Asp Asp Tyr Gly Pro
    2450                    2455                    2460

Lys Met Pro Met Val Asp Asn Leu Ala Pro Glu Leu Thr Thr Ile
    2465                    2470                    2475

Tyr Leu Glu Arg Asp Met Thr Phe Ala Val Ala Asp Glu Glu Thr
    2480                    2485                    2490

Ala Arg Thr Phe Val Ala Asp Val Glu Gly Ala Thr Ala His Phe
    2495                    2500                    2505

Asp Gly Glu Glu Trp Gln Val Thr Arg Pro Ala Gly Ala Glu Val
    2510                    2515                    2520

Arg Val Pro Arg Arg Val Ala Met Thr Arg Phe Val Gly Gly Gln
    2525                    2530                    2535

Ile Pro Gln Gly Phe Asp Pro Gly Val Tyr Gly Ile Pro Ala Asp
    2540                    2545                    2550

Met Ile Asp Asn Leu Asp Arg Val Ala Leu Trp Asn Leu Val Cys
    2555                    2560                    2565

Thr Val Asp Ala Phe Leu Ala Ala Gly Phe Ser Pro Ala Glu Leu
    2570                    2575                    2580

Leu Ala His Val His Pro Ala Arg Val Ser Ser Thr Gln Gly Thr
    2585                    2590                    2595

Gly Met Gly Gly Met Gln Ser Met Arg Ser Leu Tyr Leu Asp Lys
    2600                    2605                    2610

Leu Leu Ala Gln Pro Arg Pro Asn Asp Ile Leu Gln Glu Ala Leu
    2615                    2620                    2625

Pro Asn Val Val Ala Ala His Val Met Gln Ser Tyr Val Gly Gly
    2630                    2635                    2640

Tyr Gly Gln Met Val His Pro Val Ala Ala Cys Ala Thr Ala Ala
    2645                    2650                    2655

Val Ser Val Glu Glu Gly Met Asp Lys Leu Arg Leu Arg Lys Ala
    2660                2665                2670

Asp Phe Val Val Ala Gly Gly Ile Asp Asp Leu Ser Ile Glu Gly
    2675                2680                2685

Ile Thr Gly Phe Gly Asp Met Ala Ala Thr Ala Asp Ser Gly Glu
    2690                2695                2700

Met Glu Ala Lys Gly Ile Asp His Lys Tyr Phe Ser Arg Ala Asn
    2705                2710                2715

Asp Arg Arg Arg Gly Gly Phe Ile Glu Ser Glu Gly Gly Gly Thr
    2720                2725                2730

Ile Leu Leu Ala Arg Gly Ser Leu Ala Ala Glu Leu Gly Leu Pro
    2735                2740                2745

Val Leu Gly Val Val Gly Phe Ala Glu Ser Phe Ala Asp Gly Ala
    2750                2755                2760

His Thr Ser Ile Pro Ala Pro Gly Leu Gly Ala Leu Ser Ala Ala
    2765                2770                2775

Arg Gly Gly Ala Asp Ser Arg Leu Ala Gln Gly Leu Ala Glu Leu
    2780                2785                2790

Gly Val Ser Ala Asn Glu Ile Ala Val Ile Ser Lys His Asp Thr
    2795                2800                2805

Ser Thr Asn Ala Asn Asp Pro Asn Glu Ser Asp Leu His Glu Arg
    2810                2815                2820

Ile Ala Ala Ser Met Gly Arg Asp Ala Gly Asn Pro Leu Phe Ile
    2825                2830                2835

Ile Ser Gln Lys Thr Leu Thr Gly His Ala Lys Gly Gly Ala Ala
    2840                2845                2850

Ala Phe Gln Leu Val Gly Leu Thr Gln Val Leu Arg Asn Gly Val
    2855                2860                2865

Leu Pro Pro Asn Arg Ser Leu Asp Cys Val Asp Pro Ala Leu Arg
    2870                2875                2880

Gln His Thr His Leu Thr Trp Leu Arg Glu Gln Leu Asp Leu Arg
    2885                2890                2895

Ala Thr Pro Pro Lys Ala Gly Leu Val Thr Ser Leu Gly Phe Gly
    2900                2905                2910

His Val Ser Ala Leu Val Ala Val Val His Pro Gly Ala Phe Val
    2915                2920                2925

Glu Ala Leu Arg Ala Glu Arg Gly Ala Glu Ala Ala Ala Gln Trp
    2930                2935                2940

Gln Gln Ser Ala Asn Ala Arg Glu Ala Ala Gly Leu Gln Arg Leu
    2945                2950                2955

Thr Ser Ala Ile Ser Gly Gly Pro Ala Leu Tyr Glu Arg Pro Val
    2960                2965                2970

Asp Arg Asn Leu Gly Gly Thr Gly Asp Val Val Lys Glu Arg Glu
    2975                2980                2985

Ala Ala Val Leu Leu Asp Asp Ala Ala Arg Leu Arg Gly Ala Val
    2990                2995                3000

Leu Glu Pro Ser Thr Ser Ala Glu Ser Gly Lys Gln Gln
    3005                3010                3015

<210> 8
<211> 35
<212> PRT
<213> Artificial Sequence

<220>
<223> Linker

<400> 8

Met Ser Ala Trp Ser His Pro Gln Phe Glu Lys Gly Gly Gly Ser Gly
1               5                   10                  15

Gly Gly Ser Gly Gly Ser Ala Trp Ser His Pro Gln Phe Glu Lys Gly
            20                  25                  30

Ala Gly Ser
        35

<210> 9
<211> 15
<212> PRT
<213> Artificial Sequence

<220>
<223> Linker

<400>  9

Met Ser Ala Trp Ser His Pro Gln Phe Glu Lys Gly Ala Gly Ser
1               5                   10                  15


<210>  10
<211>  44
<212>  DNA
<213>  Artificial Sequence

<220>
<223>  Primer

<400>  10
aaaaggcgcc ggatccacta ttggcatctc taaccaccgc ctgg                        44


<210>  11
<211>  42
<212>  DNA
<213>  Artificial Sequence

<220>
<223>  Primer

<400>  11
ggtgatgatg ctcgagctgg tggcttgccg tagatcgctt gc                          42


<210>  12
<211>  27
<212>  DNA
<213>  Artificial Sequence

<220>
<223>  Primer

<400>  12
cagctcgccg gcgtcgctat ttctaag                                           27


<210>  13
<211>  30
<212>  DNA
<213>  Artificial Sequence

<220>
<223>  Primer

<400>  13
gacgccggcg agctgcgcca gcgcaataac                                        30


<210>  14
<211>  33
<212>  DNA
<213>  Artificial Sequence

<220>
<223>  Primer

<400>  14

gaaatcgtct acgccaaggg tttgaccatg cac                                    33


<210>  15
<211>  28
<212>  DNA
<213>  Artificial Sequence

<220>
<223>  Primer

<400>  15
ggcgtagacg atttctacaa cggcttcc                                         28


<210>  16
<211>  38
<212>  DNA
<213>  Artificial Sequence

<220>
<223>  Primer

<400>  16
gctcgaccca gggcacgagt atgggcggca tgcagtcg                              38


<210>  17
<211>  38
<212>  DNA
<213>  Artificial Sequence

<220>
<223>  Primer

<400>  17
cgactgcatg ccgcccatac tcgtgccctg ggtcgagc                              38


<210>  18
<211>  38
<212>  DNA
<213>  Artificial Sequence

<220>
<223>  Primer

<400>  18
cccagggcac gagttggggc ggcatgcagt cgatgcgc                              38


<210>  19
<211>  38
<212>  DNA
<213>  Artificial Sequence

<220>
<223>  Primer

<400>  19
gcgcatcgac tgcatgccgc cccaactcgt gccctggg                              38

<210>  20
<211>  30
<212>  DNA
<213>  Artificial Sequence

<220>
<223>  Primer

<400>  20
atcttgcagg aagcatatcc gaatgtcgtg                                        30


<210>  21
<211>  24
<212>  DNA
<213>  Artificial Sequence

<220>
<223>  Primer

<400>  21
tgcttcctgc aagatgtcat tcgg                                              24


<210>  22
<211>  42
<212>  DNA
<213>  Artificial Sequence

<220>
<223>  Primer

<400>  22
agaaggagat ataagcatgc tcgacaaccg tgaagcgatg ac                          42


<210>  23
<211>  38
<212>  DNA
<213>  Artificial Sequence

<220>
<223>  Primer

<400>  23
tcgagtgcgg cctaggttac cgctggtacc gcagcagg                               38